# EUROPEAN PATENT APPLICATION

(11) **EP 2 266 583 A1**
(43) Date of publication of application: **29.12.2010**
(21) Application number: 09732422.2
(22) Date of filing: 17.04.2009
(51) Int. Cl.: A61K 31/787, A61P 35/00, A61P 43/00, C07D 403/14

(54) **GENE EXPRESSION INHIBITOR SELECTIVE FOR MATRIX METALLOPROTEINASE-9 GENE**

(30) Priority: 17.04.2008 JP 2008107926
(71) Applicant: Nihon University, Tokyo 102-8275 (JP)
(72) Inventor: NAGASE, Hiroki, Tokyo 102-8275 (JP); WANG, Xiaofei, Tokyo 102-8275 (JP)
(74) Representative: HOFFMANN EITLE
(86) International application number: PCT/JP2009/057801
(87) International publication number: WO 2009/128547

(57) **Abstract**

Disclosed are: an inhibitor of the expression of matrix metalloproteinase-9 gene; a therapeutic agent for a disease associated with matrix metalloproteinase-9; and a carcinostatic agent. Each of the agents comprises a pyrrole-imidazole polyamide having an N-methylpyrrole unit, an N-methylimidazole unit and a γ-aminobutyric acid unit. The pyrrole-imidazole polyamide can be folded at the site of the γ-aminobutyric acid unit to form a U-shaped conformation in a minor groove of a double-stranded domain comprising a part or the whole of a specific nucleotide sequence (SEQ ID NO:2, SEQ ID NO:4) included in a human matrix metalloproteinase-9 gene promoter and a strand complementary to the part or the whole of the specific nucleotide sequence. In the U-shaped conformation, a Py-Im pair, an Im/Py pair and a Py/Py pair in the pyrrole-imidazole polyamide target a C-G base pair, a G-C base pair, and both of an A-T base pair and a T-A base pair in the minor groove, respectively.

SEQUENCE LISTING

<110>  Nihon University

<120>  Matrixmetalloproteinase 9 Gene Selective Expression Inhibitor

<130>  W5199-00

<150>  JP2008107926
<151>  2008-04-17

<160>  21

<170>  PatentIn version 3.3

<210>  1
<211>  700
<212>  DNA
<213>  Homo sapiens

<400>  1
tttactgccc tgaagattca gcctgcggaa gacagggggt tgccccagtg gaattcccca      60

gccttgccta gcagagccca ttccttccgc ccccagatga agcagggaga ggaagctgag      120

tcaaagaagg ctgtcaggga gggaaaaaga ggacagagcc tggagtgtgg ggaggggttt      180

ggggaggata tctgacctgg gagggggtgt tgcaaaaggc caaggatggg ccagggggat      240

cattagtttc agaaagaagt ctcagggagt cttccatcac tttcccttgg ctgaccactg      300

gaggctttca gaccaaggga tgggggatcc ctccagcttc atccccctcc ctccctttca      360

tacagttccc acaagctctg cagtttgcaa aaccctaccc ctcccctgag ggcctgcggt      420

ttcctgcggg tctggggtct tgcctgactt ggcagtggag actgcgggca gtggagagag      480

gaggaggtgg tgtaagccct ttctcatgct ggtgctgcca cacacacaca cacacacaca      540

cacacacaca cacacacaca cacaccctga cccctgagtc agcacttgcc tgtcaaggag      600

gggtggggtc acaggagcgc ctccttaaag cccccacaac agcagctgca gtcagacacc      660

tctgccctca ccatgagcct ctggcagccc ctggtcctgg                            700


<210>  2
<211>  61
<212>  DNA
<213>  Homo sapiens

<400>  2
acccctgagt cagcacttgc ctgtcaagga ggggtggggt cacaggagcg cctccttaaa      60

g                                                                      61


<210>  3
<211>  8
<212>  DNA
<213>  Homo sapiens

<400>  3
agtcagca                                                                8


<210>  4
<211>  63
<212>  DNA
<213>  Homo sapiens

<400>  4
gttgccccag tggaattccc cagccttgcc tagcagagcc cattccttcc gcccccagat      60

gaa                                                                    63


<210>  5
<211>  7
<212>  DNA

<213>  Homo sapiens

<400>  5
tggaatt                                                                 7


<210>  6
<211>  9
<212>  DNA
<213>  Homo sapiens

<400>  6
ggggtgggg                                                               9


<210>  7
<211>  7
<212>  DNA
<213>  Homo sapiens

<400>  7
tgagtca                                                                 7


<210>  8
<211>  22
<212>  DNA
<213>  Artificial

<220>
<223>  sense primer

<400>  8
gacccctgag tcagcacttg cc                                               22


<210>  9
<211>  22
<212>  DNA
<213>  Artificial

<220>
<223>  sense primer

<400>  9
gacccctgag taggcacttg cc                                               22


<210>  10
<211>  22
<212>  DNA
<213>  Artificial

<220>
<223>  sense primer

<400>  10
tgccccagtg gaattcccca gc                                               22


<210>  11
<211>  22
<212>  DNA
<213>  Artificial

<220>
<223>  sense primer

<400>  11
tgccccagtg gggttcccca gc                                               22


<210>  12
<211>  20
<212>  DNA
<213>  Artificial

<220>
<223>  forward primer

<400> 12
gagaccggtg agctggatag                                                    20


<210> 13
<211> 20
<212> DNA
<213> Artificial

<220>
<223> reverse primer

<400> 13
tacacgcgag tgaaggtgag                                                    20


<210> 14
<211> 20
<212> DNA
<213> Artificial

<220>
<223> foward primer

<400> 14
gcaccgtcaa ggctgagaac                                                    20


<210> 15
<211> 19
<212> DNA
<213> Artificial

<220>
<223> reverse primer

<400> 15
tggtgaagac gccagtgga                                                     19


<210> 16
<211> 20
<212> DNA
<213> Artificial

<220>
<223> foward primer

<400> 16
gagaccggtg agctggatag                                                    20


<210> 17
<211> 20
<212> DNA
<213> Artificial

<220>
<223> reverse primer

<400> 17
tacacgcgag tgaaggtgag                                                    20


<210> 18
<211> 20
<212> DNA
<213> Artificial

<220>
<223> forward primer

<400> 18
gcaccgtcaa ggctgagaac                                                    20


<210> 19
<211> 19
<212> DNA
<213> Artificial

<220>
<223>  reverse primer

<400>  19
tggtgaagac gccagtgga                                                    19


<210>  20
<211>  19
<212>  DNA
<213>  Artificial

<220>
<223>  forward primer

<400>  20
tgtcccttta ctgccctga                                                    19


<210>  21
<211>  23
<212>  DNA
<213>  Artificial

<220>
<223>  reverse primer

<400>  21
actccaggct ctgtcctcct ctt                                               23

## Description

### TECHNICAL FIELD

The present invention relates to matrix metalloproteinase-9 (hereinafter, also referred to as MMP-9) gene expression inhibitors, therapeutic drugs for matrix metalloproteinase-9-related disease, and anticancer agents. More specifically, the present invention relates to a drug comprising pyrrole-imidazole polyamide (hereinafter, also referred to as PIP) having a specific structure.

### BACKGROUND ART

There are findings suggesting that an environment surrounding tumors plays an exceedingly important role in cancer formation and progression. Particularly, it has been suggested that matrix metalloproteinase (hereinafter, also referred to as MMP) releases tumors from the control of their surrounding environment through the degradation or extracellular matrix to allow cell growth, invasion, dissemination and metastasis.

MMP is broadly classified into 5 groups, which have been shown to include at least 28 subtypes. Examples of their common features include: having a zinc ion binding site in the active site; requiring divalent calcium ions for activation; having a primary structure common thereto; and being produced in a latent form and activated by cleavage by themselves or other enzymes. The expression of MMP gene is broadly classified into two types: inducible and constitutive expressions. Matrix metalloproteinase-1, -3, -7, -9, -11, -12, -13, or the like, which belongs to the inducible type, is induced by tissue plasminogen activator (tPA), inflammatory cytokines, growth factors, etc., and produced mainly in fibroblasts. Although gene expression is regulated by transcription factors that act on the promoter moiety, the inducible promoter contains: a binding site for a transcription factor ETS induced by a growth factor that is not seen in a constitutive one; and a binding site TRE for a transcription factor AP-1 activated thereby. Among these MMPs, gelatinases A and B (hereinafter, also referred to as MMP-2 and MMP-9), which degrade type IV, V, VII or X collagen and fibronectin, have been reported to participate in tumor angiogenesis and also in tumor invasion, tumor metastasis or malignant transformation of tumor. It has further been suggested that TIMP-1 and TIMP-2 having inhibitory effect on gelatinase also participate in cancer as endogenous inhibitors of MMP. A large number of reports have showed the high expressions of MMP-2 and MMP-9 in tumors, particularly, in highly malignant cancer. It has been demonstrated in cultured cells and *in vivo* that MMP-9 plays an important role in tumor invasion and angiogenesis (NON-PATENT DOCUMENTS 1 and 2).

92-kDa type IV collagenase MMP-9 (or also called gelatinase B), after being expressed on the cell surface of host macrophage, promoted the growth and invasion of xenotransplanted cancer cells, while such growth and invasion were suppressed in MMP-9-knockout host macrophage that does not express MMP-9. Moreover, BPHA (MMP inhibitor), which specifically inhibits MMP-2, MMP-9 or MMP-14, suppressed angiogenesis in cancer cell-transplanted subcutaneous chambers.

MMP-9-knockout (-/-) mice injected with human ovarian cancer cells exhibited tumor incidence and tumor growth statistically significantly lower than those of MMP-9 wild-type (+/+) mice. Moreover, melanoma cell strains at the early stage lack MMP-9 expression, and not only MMP-9 expression but also a large amount of MMP-9 secretion is induced in these cells after induction by tumor growth factor (TGF), tumor necrosis factor (TNF), interleukin- 1 (IL-1) and tissue plasminogen activator (tPA). These facts and inhibitory effects on tumor or inflammation in experimental animals have suggested that the dramatic expression of MMP-9 in large amounts in cancer plays an important role in the latent invasion and metastasis of the cancer cells. Thus, it has been suggested that MMP-9 is a potential target of new drugs. Based on such findings, MMP inhibitors have been developed and clinically tested. However, the development of MMP inhibitors lags behind on the private-sector level due to the failure of the clinical test of an MMP inhibitor marimastat (BB-2516).

This marimastat is an extensive metalloproteinase inhibitor (metalloenzyme inhibitor that inhibits a wide range of metalloenzymes) and had failed to reach actual use due to adverse reactions such as myalgia and arthralgia found in the clinical test. A large number of MMP activity inhibitors had been prepared to go to clinical tests, which were, however, postponed due to adverse reactions, as in marimastat. Since MMPs have common features structurally or in property, small molecules of specific inhibitors for single subtypes are difficult to develop. Moreover, recent studies have revealed that some MMPs are located within cells and also involved in the degradation of intracellular proteins. The adverse reaction problem attributed to the simple inhibition of MMPs may not be avoided. However, there may still remain the possibility that the adverse reaction problem will be minimized or decreased to a level that does not matter clinically, if an inhibitor appropriately knocks down only particular MMP in a disease cell-specific manner and, furthermore, is used in a limited site.

Pyrrole-imidazole polyamide is a synthetic chemical that was found by Dervan et al., based on the fact that antibiotics duocarmycin-A and distamycin-A recognize DNA in a base-specific manner (PATENT DOCUMENT 1 and NON-PATENT DOCUMENTS 3 and 4). PIP can specifically regulate the expression of a target gene, because it recognizes double-stranded DNA in a nucleotide sequence-specific manner and binds to a minor groove in the DNA double helix structure (NON-PATENT DOCUMENT 4). Moreover, PIP, unlike previous gene expression regulators such as antisense/ribozyme/siRNA, is not degraded by nuclease *in vivo* and is highly capable of binding to nucleic acids. Thus, its clinical application to anticancer agents or the like as a novel molecular target therapeutic drug is expected.

The approach of inactivating gene functions by reverse genetics is used for analyzing the functions of a certain gene. On the other hand, it also opens up great possibilities for treating viral infection, cancer and other diseases based on the abnormal expression of genes. Specifically, it has been known that the inactivation of gene functions can be carried out at the DNA level by homologous recombination or at the RNA level using antisense oligodeoxynucleotide or ribozyme. However, the homologous recombination had problems of low recombination efficiency in general and effectiveness limited to some cells, while the approach using antisense oligodeoxynucleotide or ribozyme had problems of limitations on targeted sequences, poor migration into tissues or cells, and susceptibility to ribonuclease degradation.

On the other hand, it has been reported that pyrrole-imidazole polyamide, unlike antisense reagents or (deoxy)ribonucleotide reagents (e.g., ribozyme), can specifically recognize the nucleotide sequence of DNA and extracellularly control the expression of the particular gene.

The pyrrole-imidazole polyamide (hereinafter, also referred to as Py-Im polyamide) is a group of synthetic small molecules and comprises an aromatic ring N-methylpyrrole unit (hereinafter, also referred to as Py) and an N-methylimidazole unit (hereinafter, also referred to as Im) (PATENT DOCUMENT 1 and NON-PATENT DOCUMENT 3). These Py and Im units can be continuously coupled and folded to form a U-shaped conformation in the presence of γ-aminobutyrate. In the pyrrole-imidazole polyamide according to the present invention, the N-anethylpyrrole unit (Py), the N-methylimidazole unit (Im) and the γ-aminobutyrate unit (also referred to as a γ linker) are linked to each other via aide bond (-C(=O)-NH-), and its general structure and production method are known in the art (PATENT DOCUMENTS 2 to 4).

Such synthetic polyamide can bind with high affinity and specificity to particular base pairs in a minor groove of double helix DNA. This specific recognition of base pairs depends on the one-to-one pair formation of Py and/or Im. Specifically, in the U-shaped conformation in the minor groove of DNA, a Py/Im pair targets a C-G base pair, Im/Py targets a G-C base pair, and Py/Py targets both an A-T base pair and a T-A base pair (NON-PATENT DOCUMENT 3). Recent studies have demonstrated that the A-T condensation can be overcome by the preferential binding of Hp/Py to a T/Apair, as a result of substituting one pyrrole ring in the Py/Py pair with 3-hydroxypyrrole (Hp).

In general, the initiation of transcription is regarded as being an important point of gene control. The transcription initiation requires some processes in which transcription factors binding to specific recognition sequences in a gene promoter region form a complex, which then binds to the DNA, sequence. The polyamide in the minor groove may interfere with gene regulation by blocking the binding of a transcription factor or a complex, if the binding of the transcription factor or the complex to the particular sequence is important for the gene expression. This hypothesis has been verified by *in-vitro* and *in-vivo* experiments. An 8-membered ring Py-Im polyamide bound within a zinc finger recognition site (TFIIIA binding site) inhibited the transcription of the 5S RNAgene. Polyamides that bind to base pairs adjacent to transcription factor sequences in a human immunodeficiency virus type 1 (HIV-1) promoter inhibit HIV-1 replication in human cells. These sequences encompass TATA box, lymphocyte enhancer factor LEF-1 sequence and ETS-1 sequence. In contrast to this, polyamide also activates gene expression by blocking a repressor factor or replacing an original transcription factor. UL122-mediated early protein 2 (IE86) of human cytomegalovirus (CMV) blocks the supply of RNA polymerase II to the promoter and inhibits the transcription of the related genes. Synthetic polyamide can block the inhibition by IE86 and release the expression of the corresponding gene. Polyamide designed by Mapp et al. acts as an artificial transcription factor and mediates gene transcription reaction.

### CITATION LISTS

### PATENT DOCUMENTS

PATENT DOCUMENT 1: WO98/49142 A1
PATENT DOCUMENT 2: Japanese Patent No. 3045706
PATENT DOCUMENT 3: JP 2001-136974 A
PATENT DOCUMENT 4: WO03/000683 A1

### NON-PATENT DOCUMENTS

NON-PATENT DOCUMENTS 1: Katori et al: J. Surg. Oncol. 93, 2006, 80-85
NON-PATENT DOCUMENT 2: Lakka et al: J. Biol. Chem. 280 (23), 2005,21882-21892
NON-PATENT DOCUMENT 3: Sugiyama et al: Proc Natl Acad Sci USA. 1996; 93: 14405-14410
NON-PATENT DOCUMENT 4: Dervan: Bioorg Med. Chem. 2001; 9: 2215-35

### SUMMARY OF THE INVENTION

### PROBLEMS TO BE SOLVED BY THE INVENTION

The approach using antisense oligodeoxynucleotide or ribozyme described above had problems of limitations on targeted sequences, poor migration into tissues or cells, and susceptibility to ribonuclease degradation. There has been no report so far on a matrix metalloproteinase-9 gene expression inhibitor or a therapeutic drug for matrix metalloproteinase-9-related disease, comprising pyrrole-imidazole polyamide binding to the nucleotide sequence of matrix metalloproteinase-9 gene.

### MEANS FOR SOLVING THE PROBLEMS

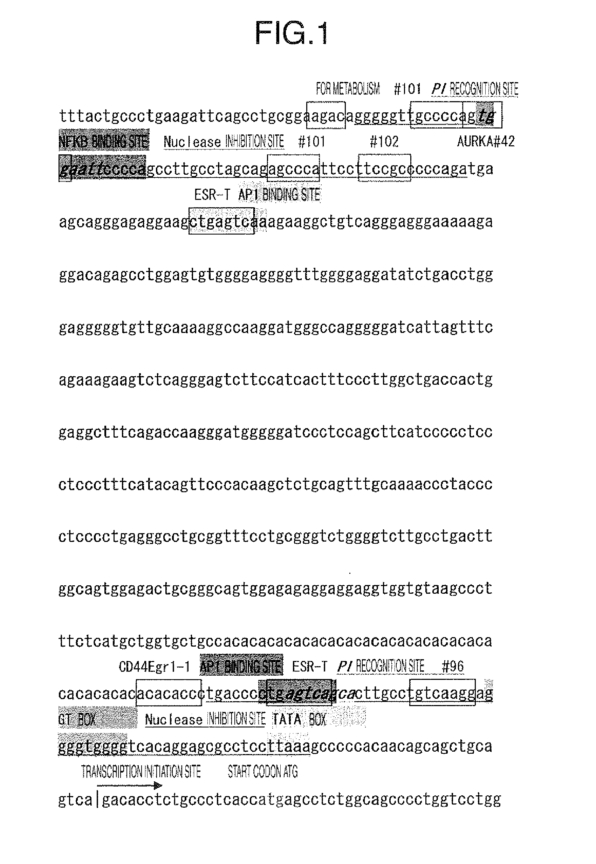

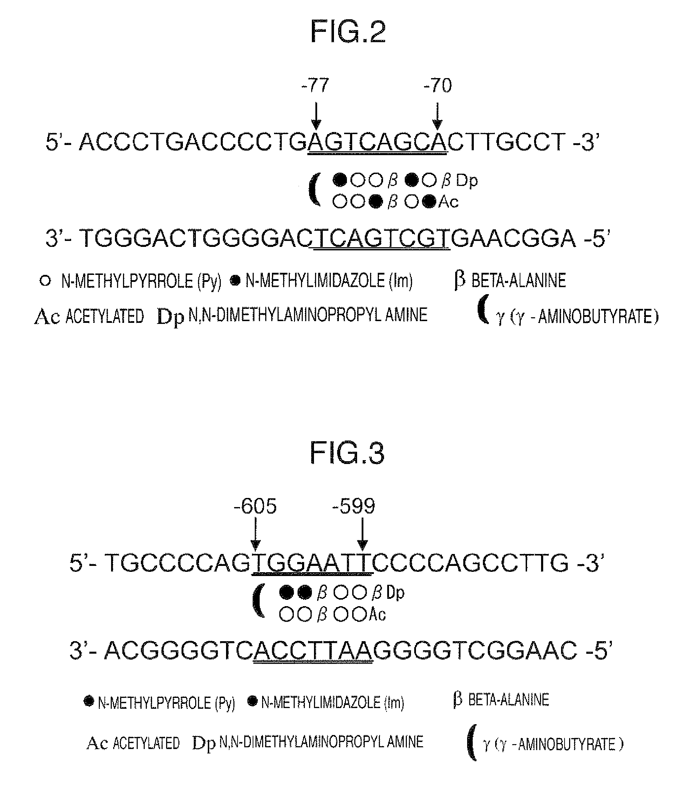

The present inventors have conducted diligent studies on the development of pyrrole-imidazole polyamides that can specifically bind to a particular regions in a matrix metalloproteinase-9 (MMP9) promoter and inhibit the expression of the matrix metalloproteinase-9 gene, and on its pharmacological effect. Thus, to obtain a compound that can inhibit the expression of human matrix metalloproteinase-9 (hereinafter, also referred to as hMMP-9) gene and is useful as a therapeutic drug, the present inventors have prepared polyamides targeting various fragments of the matrix metalloproteinase-9 gene promoter and consequently found that a compound that binds to a region containing an AP-1 binding region and a GT box control region, preferably a region from -84 to -24 (SEQ ID NO: 2), more preferably a region from -77 to -70 (Fig. 2, SEQ ID NO: 3), or a region containing an NF-κB binding region, preferably a region from -615 to -553 (SEQ ID NO: 4), more preferably a region from -605 to -599 (Fig. 3, SEQ ID NO: 5), in a region from -653 to -24 in the upstream promoter control region up to 653 bases of the MMP-9 gene or a sequence represented by Fig. 1 or SEQ ID NO: 1, significantly inhibits the activity of the human MMP-9 gene promoter and down-regulates the expression of the human MMP-9 gene in human breast cancer MDA-MB-231 cells. Based on these findings, the present invention has been completed.

Specifically, the present invention is as follows:
(1) A medicament comprising pyrrole-imidazole polyamide containing an N-methylpyrrole unit, an N-methylimidazole unit and a γ-aminobutyrate unit, wherein the pyrrole-imidazole polyamide can be folded at the γ-aminobutyrate unit into a U-shaped conformation in a minor groove of a double helix region (hereinafter, referred to as a target region) which comprises a portion or the whole of a nucleotide sequence from -84 to -24 (SEQ ID NO: 2) in a human matrix metalloproteinase-9 gene promoter, and a strand complementary thereto, wherein a Py/Im pair corresponds to a C-G base pair, an Im/Py pair corresponds to a G-C base pair, and a Py/Py pair corresponds to both an A-T base pair and a T-A base pair.
(2) The medicament according to (1), further comprising a β-alanine unit.
(3) The medicament according to (1), further comprising a fluorescein isothiocyanate (hereinafter, also referred to as FITC) unit.
(4) The medicament according to any one of (1) to (3), wherein the drug is intended for inhibition of human matrix metalloproteinase-9 gene expression.
(5) The medicament according to any one of (1) to (3), wherein the drug is intended for treatment of human matrix metalloproteinase-9-related disease.
(6) The medicament according to (5), wherein the drug is intended for use as an anticancer agent.
(7) The medicament according to any one of (1) to (6), wherein the target region is a double helix region which comprises a portion or the whole of a nucleotide sequence from - 77 to -70 (SEQ ID NO; 3) in the human matrix metalloproteinase-9 promoter, and a strand complementary thereto.
(8) The medicament according to any one of (1) to (7), wherein the pyrrole-imidazole polyamides is represented by the following formula: 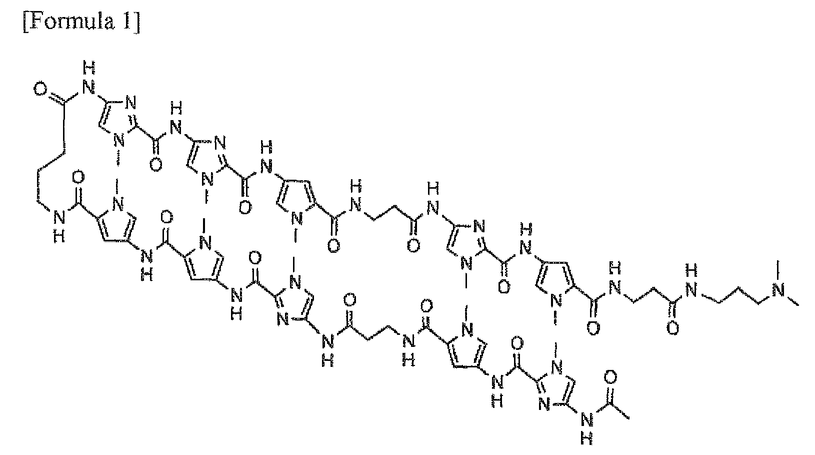
(9) Pyrrole-imidazole polyamide represented by the following formula: 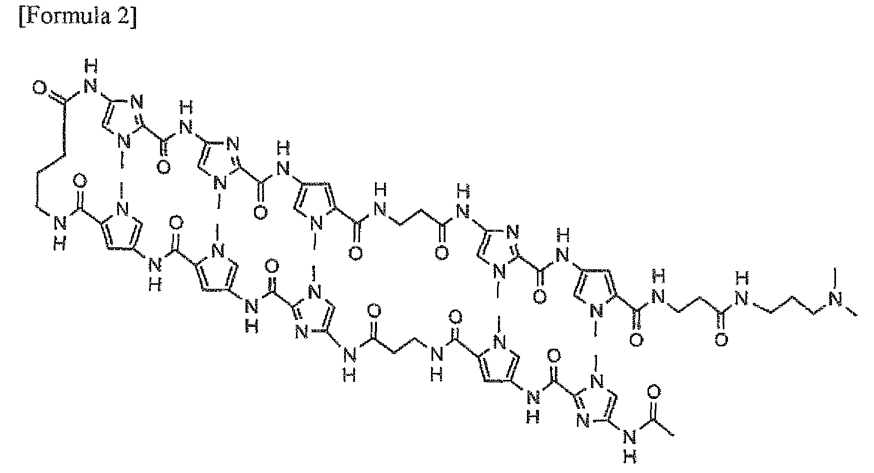
(10) A medicament comprising pyrrole-imidazole polyamide containing an N-methylpyrrole unit, an N-methylimidazole unit and a γ-aminobutyrate unit, wherein the pyrrole-imidazole polyamide can be folded at the γ-aminobutyrate unit into a U-shaped conformation in a minor groove of a double helix region which comprises a portion or the whole of a nucleotide sequence from -615 to -553 (SEQ ID NO: 4) in a human matrix metalloproteinase-9 gene promoter, and a strand complementary thereto, wherein a Py/Im pair corresponds to a C-G base pair, an Im/Py pair corresponds to a G-C base pair, and a Py/Py pair corresponds to both an A-T base pair and a T-A base pair.
(11) The medicament according to (10), further comprising a β-alanine unit.
(12) The medicament according to (10), further comprising a fluorescein isothiocyanate (hereinafter, also referred to as FITC) unit.
(13) The medicament according to any one of (10) to (12), wherein the drug is intended for inhibition of human matrix metalloproteinase-9 gene expression.
(14) The medicament according to any one of (10) to (12), wherein the drug is intended for treatment of human matrix metalloproteinase-9-related disease.
(15) The medicament according to (14), wherein the drug is intended for use as an anticancer agent.
(16) The medicament according to any one of (10) to (15), wherein the target region is a double helix region which comprises a portion or the whole of a nucleotide sequence from -605 to -599 (SEQ ID NO: 5) in the human matrix metalloproteinase-9 promoter, and a strand complementary thereto.
(17) The medicament according to any one of (10) to (16), wherein the pyrrole-imidazole polyamide is represented by the following formula: 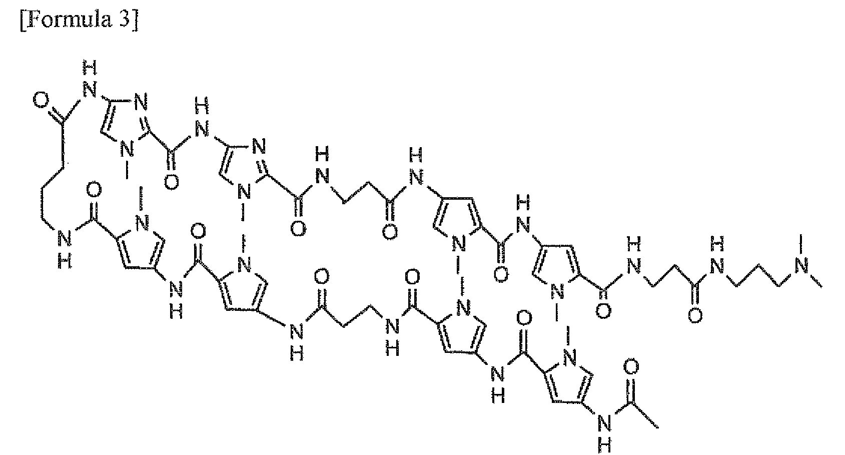
(18) Pyrrole-imidazole polyamide represented by the following formula: 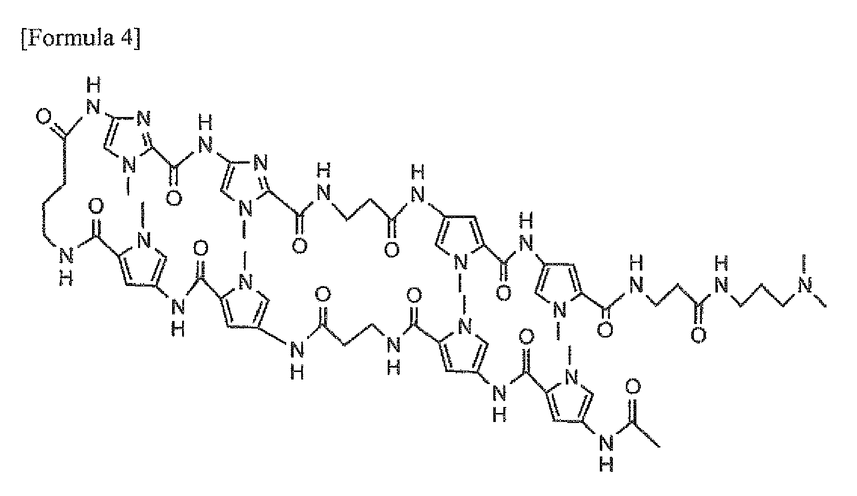
(19) A pharmaceutical composition comprising a drug according to any one of (1) to (8) or a drug according to any one of (10) to (17) and a pharmaceutically acceptable carrier.
(20) A medicament comprising pyrrole-imidazole polyamide according to (9) or (18) and a pharmaceutically acceptable carrier.
(21) The medicament according to any one of (1) to (8), wherein a terminal carboxyl group of the pyrrole-imidazole polyamide forms amide.
(22) The medicament according to (21), wherein the amide is amide with methylaminopropyl amine or N,N-dimethylaminopropyl amine.
(23) The medicament according to any one of (10) to (17), wherein a terminal carboxyl group of the pyrrole-imidazole polyamide forms amide.
(24) The medicament according to (23), wherein the amide is amide with methylaminopropyl amine or N,N-dimethylaminopropyl amine.
(25) Pyrrole-imidazole polyamide represented by the following formula: 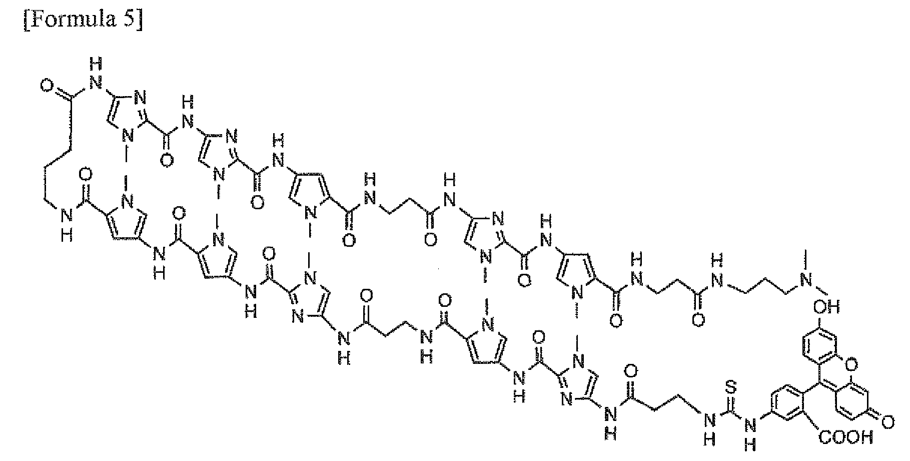
(26) Pyrrole-imidazole polyamide represented by the following formula: 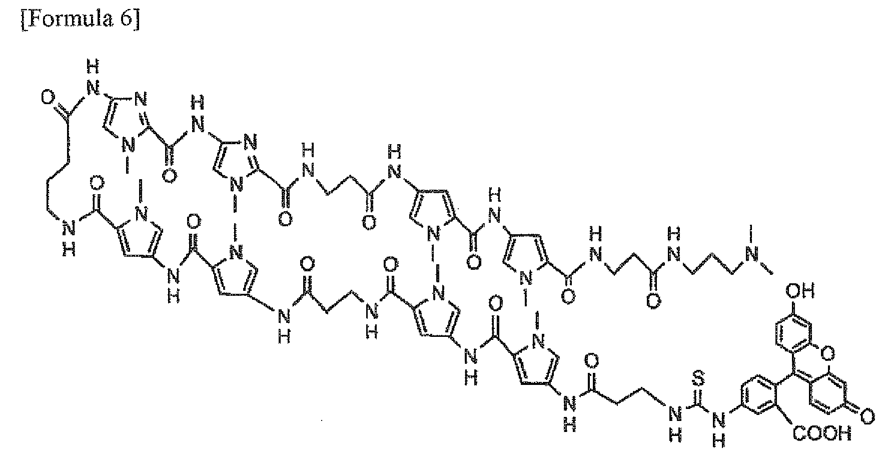
(27) The pyrrole-imidazole polyamide according to (9), (18), (25) or (26), wherein the pyrrole-imidazole polyamide is used as a reagent of a basic experiment.

### ADVANTAGE OF THE INVENTION

According to the present invention, a drug for inhibition of matrix metalloproteinase-9 gene expression, a drug for treatment of matrix metalloproteinase-9 gene-related disease, and an anticancer agent can be obtained, which are free from adverse reactions as in chemotherapeutic agents because of being capable of specifically inhibiting gene expression and are free from disadvantageous degradation by ribonuclease because of being compounds. Furthermore, according to the present invention, a reagent of a basic experiment using this gene can be obtained.

### BRIEF DESCRIPTION OF THE DRAWINGS

Fig. 1 shows the nucleotide sequence of a human matrix metalloproteinase-9 gene promoter region;
Fig. 2 shows a binding site, on the human MMP-9 promoter sequence, of pyrrole-imidazole polyamide HMMP9AP1 of the present invention;
Fig. 3 shows a binding site, on the human MMP-9 promoter sequence, of pyrrole-imidazole polyamide HMMP9NFκβ of the present invention;
Fig. 4 shows pyrrole-imidazole polyamide HMMP9AP1 of the present invention;
Fig. 5 shows pyrrole-imidazole polyamide HMMP9AP1-FITC of the present invention;
Fig. 6 shows pyrrole-imidazole polyamide HMMP9NFκβ of the present invention;
Fig. 7 shows pyrrole-imidazole polyamide HMMP9NFκβ-FITC of the present invention;

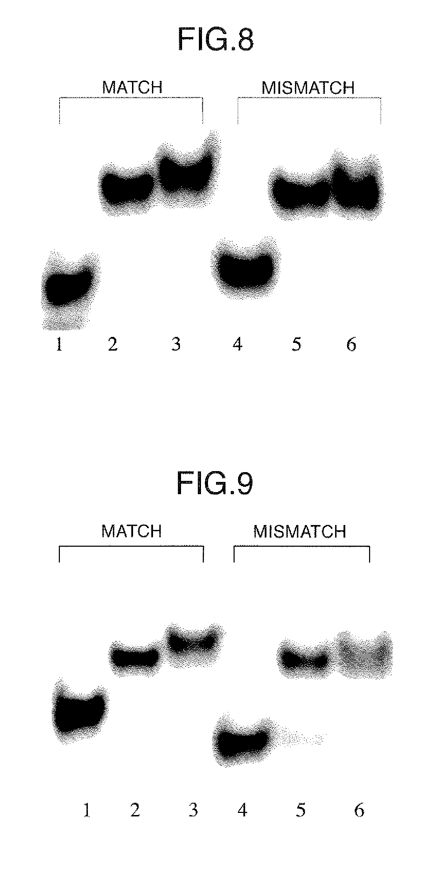

Fig. 8 shows results of gel shift analysis (EMSA) using HMMP9AP1. Lane 1 shows the gel shift analysis results of "match" single-stranded DNA, lane 2 shows the gel shift analysis results of "match" double-stranded DNA, lane 3 shows the gel shift analysis results of HMMP9AP1-supplemented "match "double-stranded DNA, lane 4 shows the gel shift analysis results of "mismatch" single-stranded DNA having 2-bp mutation, lane 5 shows the gel shift analysis results of "mismatch" double-stranded DNA having 2-bp mutation, and lane 6 shows the gel shift analysis results of HMMP9AP1-supplemented "mismatch" double-stranded DNA having 2-bp mutation;
Fig. 9 shows results of gel shift analysis (EMSA) using HMMP9NFκβ. Lane 1 shows the gel shift analysis results of "match" single-stranded DNA, lane 2 shows the gel shift analysis results of "match" double-stranded DNA, lane 3 shows the gel shift analysis results of HMhF9NFκβ-supplemented "match "double-stranded DNA, lane 4 shows the gel shift analysis results of "mismatch" single-stranded DNA having 2-bp mutation, lane 5 shows the gel shift analysis results of "mismatch" double-stranded DNA having 2-bp Mutation, and lane 6 shows the gel shift analysis results of HMMP9NFκβ-supplemented "mismatch" double-stranded DNA having 2-bp mutation;
Fig. 10 shows results ofwound-healing migration assay. These results show that the cellular migration of MDA-MB-231 cells treated with HMMP9AP 1 decreases in a concentration-dependent manner;
Fig. 11 shows results of wound-healing migration assay. These results show that the cellular migrations of MDA-MB-231 cells and HeLa cells treated with HMMP9NFκβ decrease in a concentration-dependent manner;
Fig. 12 shows results of invasion assay into matrigel. This drawing shows results of *in-vitro* invasion, into matrigel, of MDA-MB-231 cells treated with HMMP9AP1. These results show that the invasion into matrigel decreases in an HMMP9AP1 concentration-dependent manner. The results are indicated in mean±SE from three experiments;
Fig. 13 shows results of invasion assay into matrigel. This drawing shows results of *in-vitro* invasions, into matrigel, of HeLa cells and MDA-MB-231 cells treated with HMMP9NFκβ. These results show that the invasion into matrigel decreases in an HMMP9NFκβ concentration-dependent manner. The results are indicated in mean±SE from three experiments;
Fig. 14 shows results of real-time RT-PCR. These results show that treatment with HMMP9AP1 decreases the expression level of mRNA in MDA-MB-231 cells in a concentration-dependent manner. The results are indicated in mean±SE from three experiments. When the p-value is P<0.05(*), the results were determined to be significant;
Fig. 15 shows results of real-time RT-PCR. These results show that treatment with HMMP9NFκβ decreases the expression level of mRNA in MDA-MB-231 cells in a concentration-dependent manner. The results are indicated in mean±SE from three experiments. When the p-value is P<0.05(*), the results were determined to be significant;
Fig. 16 shows results of zymography assay using gelatin as a substrate. A conditioned medium obtained from MDA-MB-231 cells treated with HMMP9AP1 was electrophoresed on a 10% zymogram gelatin gel. A distinct band on the gel depicts the activity of matrix metalloproteinase;
Fig. 17 shows results of zymography assay using gelatin as a substrate. A conditioned medium obtained from MDA-MB-231 cells treated with HMMP9NFκβ was electrophoresed on a 10% zymogram gelatin gel. A distinct band on the gel depicts the activity of matrix metalloproteinase;
Fig. 18 shows results of western blotting analysis (protein assay). This drawing shows the western blotting results of proteins extracted from MDA-MB-231 cells treated with HMMP9NFκβ. It also shows results of reprobing with β-actin after stripping of a PVDF (polyvinylidene difluoride) membrane probed with antibodies against MMP-9 protein (lower diagram). These results show that the expression level of MMP-9 protein decreases in a concentration-dependent manner;
Fig. 19 shows the distribution of fluorescently labeled PIP. This drawing shows the intracellular distribution of HMMP9AP 1 after 45-minutes incubation of cells supplemented with fluorescently labeled HMMP9AP1. It shows the results obtained using bright field (Fig.19a), fluorescence (Fig. 19b), Hoechst 33342 (Fig. 19c) and Merge (Fig. 19d). Hoechst 33342 stains the nuclei;
Fig. 20 shows the distribution of fluorescently labeled PIP. This drawing shows the intracellular distribution of HMMP9NFκβ after 30-minute or 96-hour incubation of cells supplemented with fluorescently labeled HMMP9NFκβ. It shows the results obtained using bright field (Fig. 20a), Hoechst 33342 (Fig. 20b) and fluorescence (Fig. 20c). Hoechst 33342 stains the nuclei;
Fig. 2 shows or results of HPLC of HMMP9AP1 of the present invention;

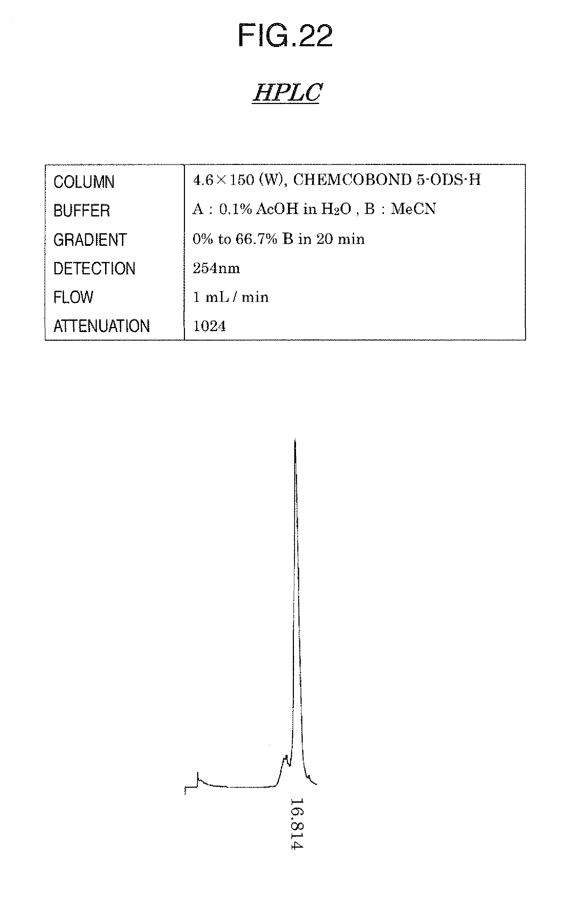

Fig. 22 shows results of HPLC of HMMP9AP1-FITC of the present invention;

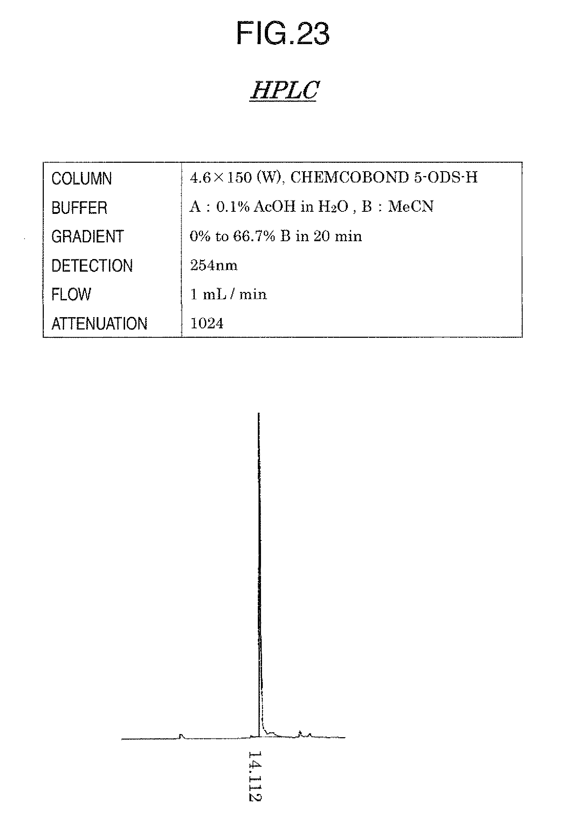

Fig. 23 shows results of PLC of HMMP9NFκβ of the present invention;

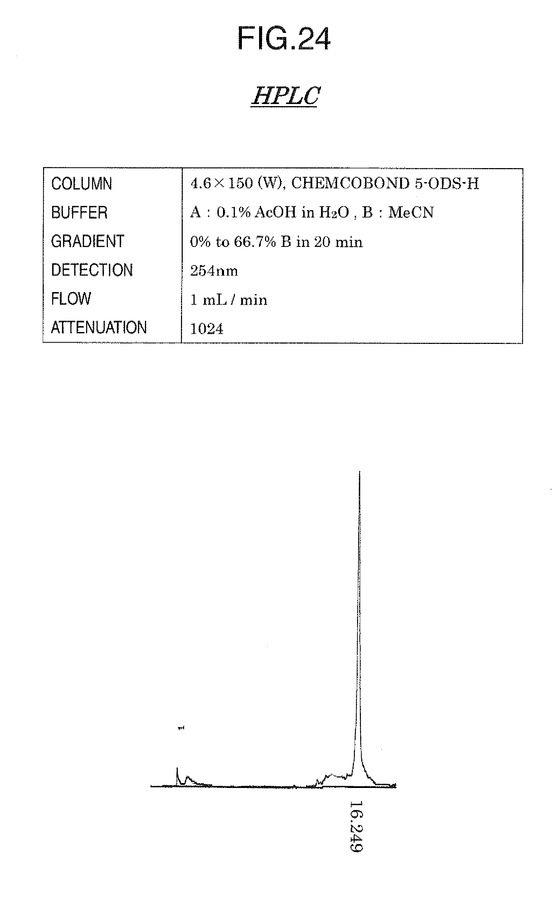

Fig. 24 shows results of HPLC of HMMP9NFκβ-FITC of the present invention;

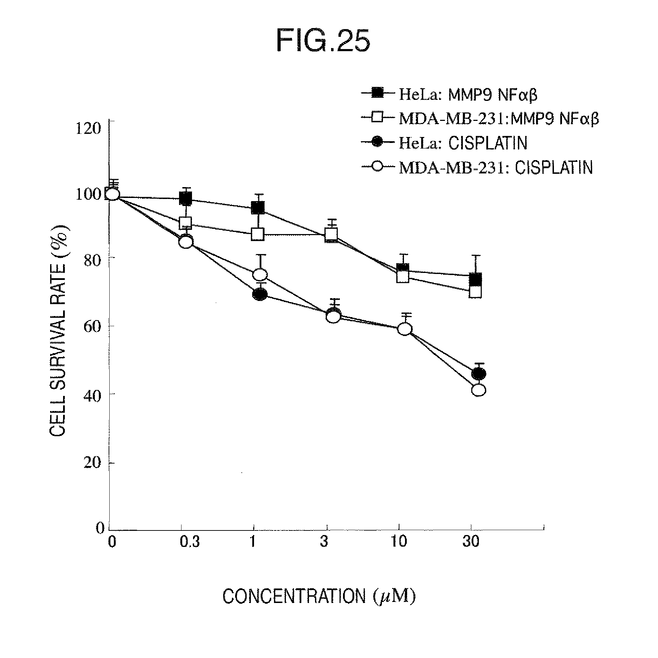

Fig. 25 shows results of growth inhibition assay using MDA-MB-231 cells and HeLa cells. These results show that the growths of the MDA-MB-231 cells and HeLa cells were inhibited by HMMP9NFκβ of the present invention;

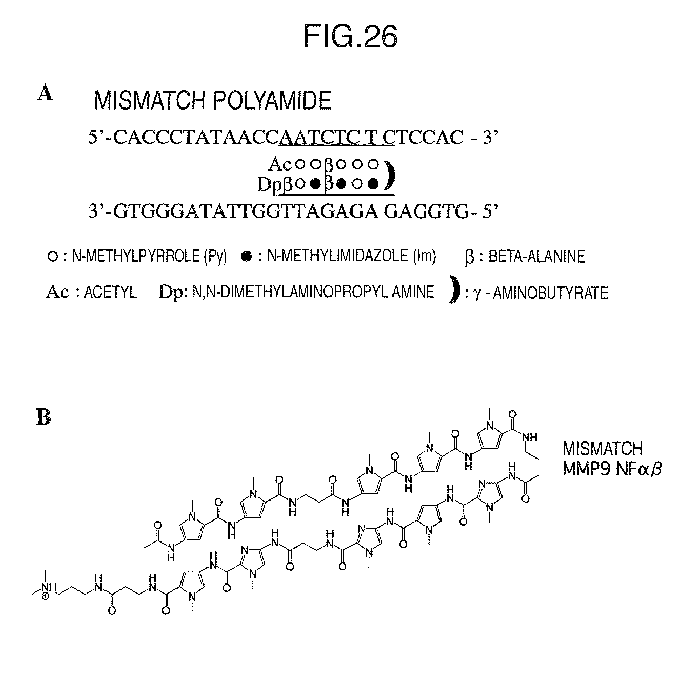

Fig. 26 shows a binding sequence of mismatch pyrrole-imidazole polyamide (Fig. 26A) and mismatch pyrrole-imidazole polyamide;

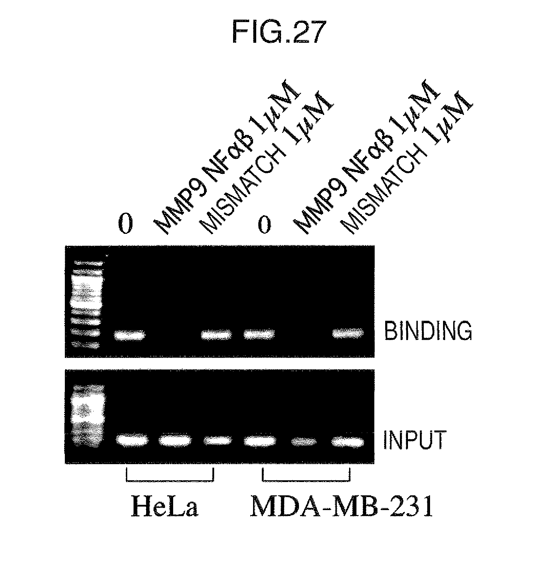

Fig. 27 shows results of immunoprecipitation/PCR assay with anti-NF-κβ antibodies. These results show that the polyamide MMP9NFκβ of the present invention inhibited *in vivo* the specific binding of NF-κβ protein with a MMP-9 promoter region;

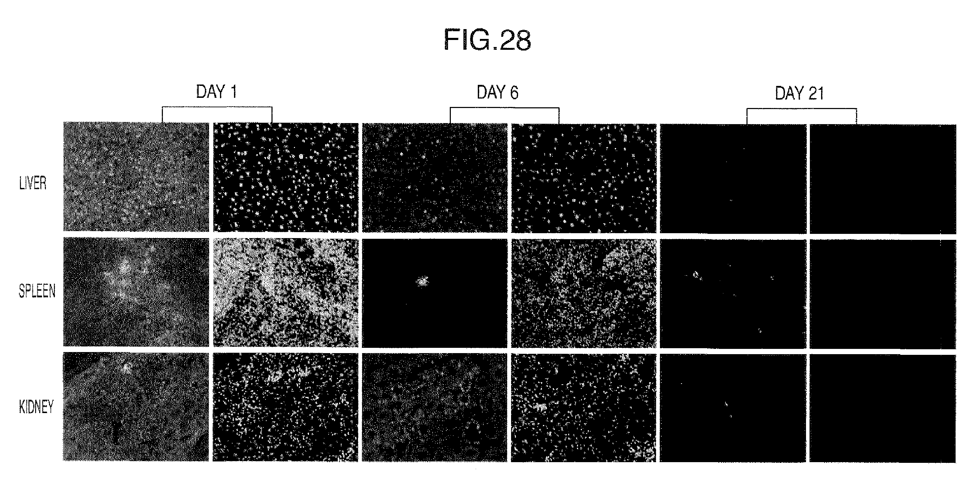

Fig. 28 shows the tissue distribution of fluorescently labeled polyamide HMMP9NFκβ. A large number of FITC-labeled polyamides HMMP9NFκβ were incorporated after 1 day of administration into the nuclei of cells in the liver and the kidney. Moreover, their incorporation was also observed in the nuclei of cells in the spleen. Even after 6 days from addition, strong FITC fluorescence was observed; and

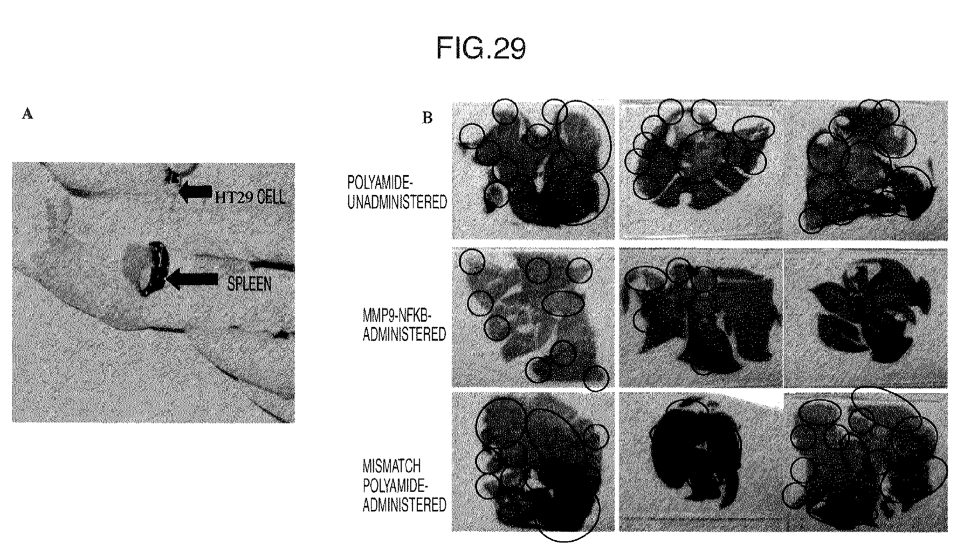

Fig. 29 shows results of evaluation of polyamide HMMP9NFκβ of the present invention using immunodeficient athymic mouse (nude mouse) models with liver metastasis from human colon cancer. The HMMP9-NFκβ polyamide inhibited the liver metastasis of human colon cancer transplanted in the spleens of the nude mice.

### BEST MODE FOR CARRYING OUT THE INVENTION

In pyrrole-imidazole polyamide according to the present invention, an N-methylpyrrole unit, an N-methylimidazole unit and a γ-aminobutyrate unit (hereinafter, also referred to as a γ linker) are linked to each other via amide bond (-C(=O)-NH-), and its general structure and production method are known in the art (see e.g., PATENT DOCUMENTS 1 to 3).

The pyrrole-imidazole polyamide can be produced, for example, by an automatic synthesis method based on solid-phase method using Fmoc (9-fluorenylmethoxycarbonyl) (solid-phase Fmoc method) (PATENT DOCUMENT 3). According to the solid-phase Fmoc method, the pyrrole-imidazole polyamide can be excised as a terminal carboxylic acid residue from the solid carrier. Therefore, various functional groups can be introduced to the terminus of the molecule to prepare derivatives of the pyrrole-imidazole polyamide. For example, compounds capable of alkylating DNA, such as duocarmycin, pyrrolobenzodiazepine, bleomycin, enediyne compounds, nitrogen mustard and derivatives thereof can also be introduced, if necessary.
Since the solid-phase Fmoc method is an automatic synthesis method using a commercially available protein (peptide) synthesizer, conjugates of the pyrrole-imidazole polyamide with a naturally-occurring or non-natural protein can also be synthesized. Moreover, since the Fmoc method is performed under milder reaction conditions than those of t-BOC method, organic compounds other than proteins (including even compounds having functional groups that are unstable under acidic conditions) may be introduced. For example, conjugates of the pyrrole-imidazole polyamide with DNA or RNA (or a derivative thereof) may also be synthesized automatically.

According to the Fmoc method or the like known in the art, pyrrole-imidazole polyamide having a terminal carboxyl group can be synthesized. Specific examples thereof include pyrrole-imidazole polyamide having a β-alanine residue (β-aminopropionate residue) or a γ-aminobutyrate residue at the terminus. The pyrrole-imidazole polyamide having a β-alanine residue or a γ-aminobutyrate residue at the terminus can be synthesized, for example, by the solid-phase Fmoc method using a peptide synthesizer with a solid-phase carrier holding aminopyrrolecarboxylic acid, aminoimidazolecarboxylic acid and β-alanine or γ-aminobutyrate, the amino groups of which are respectively protected with Fmoc.

Specific examples of the aminopyrrolecarboxylic acid include 4-amino-2-pyrrolecarboxylic acid, 4-amino-1-methyl-2-pyrrolecarboxylic acid, 4-amino-1-ethyl-2-pyrrolecarboxylic acid, 4-amino-1-propyl-2-pyrrolecarboxylic acid and 4-amino-1-butyl-2-pyrrolecarboxylic acid. Specific examples of the aminoimidazolecarboxylic acid include 4-amino-2-imidazolecarboxylic acid, 4-amino-1-methyl-2-imidazolecarboxylic acid, 4-amino-1-ethyl-2-imidazolecarboxylic acid, 4-amino-1-propyl-2-imidazolecarboxylic acid and 4-amino-1 butyl-2-imidazolecarboxylic acid.

According to the solid-phase Fmoc method, for examples, conjugates of the pyrrole-imidazole polyamide and FITC (fluorescein isothiocyanate) can also be synthesized. FITC has heretofore been known as a fluorescent labeling reagent for antibodies. Accordingly, the obtained conjugates can be used for demonstrating that this pyrrole-imidazole polyamide recognizes a particular DNA sequence.

A matrix metalloproteinase-9 gene expression inhibitor of the present invention comprises pyrrole-imidazole polyamide containing an N-methylpyrrole unit (Py), an N-methylimidazole unit (Im) and a γ-aminobutyrate unit, wherein the pyrrole-imidazole polyamide can be folded at the γ-aminobutyrate unit into a U-shaped conformation in a minor groove of a double helix region (hereinafter, referred to as a target region) which comprises a portion or the whole of a nucleotide sequence from -77 to -70 (SEQ ID NO: 3) or a nucleotide sequence from - 605 to -599 (SEQ ID NO: 5) in a human matrix metalloproteinase-9 promoter, and a strand complementary thereto, wherein a Py/Im pair corresponds to a C-G base pair, an Im/Pγ pair corresponds to a G-C base pair, and a Py/Py pair corresponds to both an A-T base pair and a T-A base pair.

The backbone of DNA helix usually forms 2 kinds of grooves, a wide and deep groove of which is called a major groove and a narrow and shallow groove of which is called a minor groove. In this context, the pyrrole-imidazole polyamide can bind via non-conjugated bond with high affinity and specificity to the minor groove formed by particular base pairs. In this binding, the Py/Im pair, Im/Py pair, and Py/Py pair of the pyrrole-imidazole polyamide correspond to the C-G base pair, G-C base pair, and both A-T and T-A base pairs of the minor groove, respectively. Furthermore, the pyrrole-imidazole polyamide molecule is folded at the intramolecular γ-aminobutyrate unit to form a U-shaped conformation.

When a base pair in the minor groove does not correspond to a pair formed by Py and/or Im of the pyrrole-imidazole polyamides, the binding of the pyrrole-imidazole polyamide to the minor groove will be insufficient. In the present specification, such pyrrole-imidazole polyamide whose pair formed by Py and/or Im does not correspond to a base pair in the minor groove is called a mismatch or a mismatch polyamide.

The nucleotide sequence of the human matrix metalloproteinase-9 gene control region is as shown in Fig. 1 and SEQ ID NO: 1.
Pyrrole-imidazole polyamides HMMP9AP1 and HMMP9NFκβ of the present invention are as shown below.

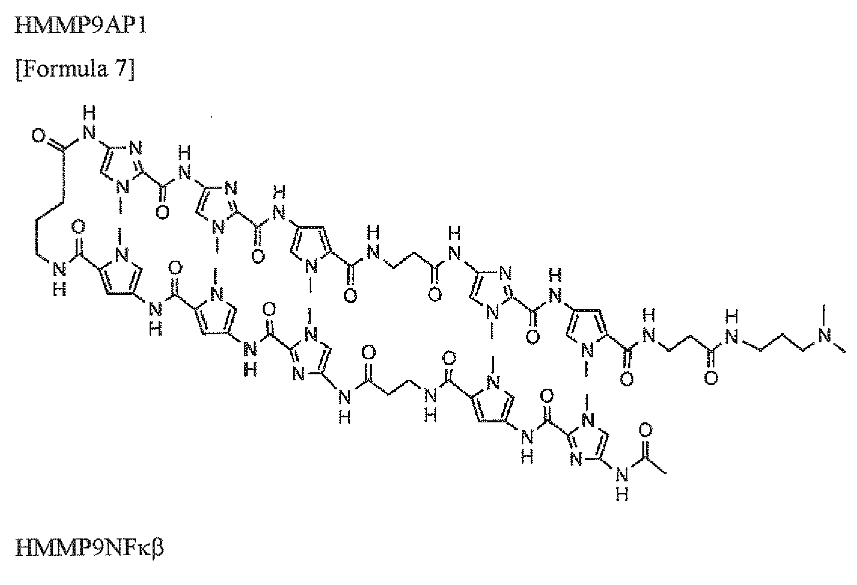

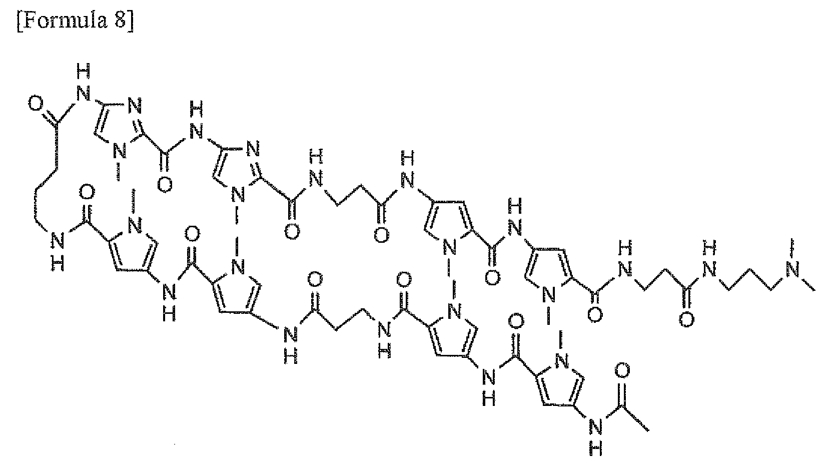

The HMMP9AP1 is represented by the molecular formula C₇₅H₉₃N₃₁O₁₅ with a molecular weight of 1668.6, and its target sequence is a region from -84 to -24 (SEX ID NO: 2) containing an AP 1 binding region and a GT box control region, in the region from -653 to -24 of the human matrix metalloproteinase-9 gene control region (SEQ ID NO: 1). More specifically, the HMMP9AP1 inhibits the expression of the human matrix metalloproteinase-9 gene by binding to an 8-base region from -77 to -70 (agtcagca; SEQ ID NO: 3).

The HMNW9NFκβ is represented by the molecular formula C₆₆H₈₄N₂₄O₁₃ with a molecular weight of 1421.3, and its target sequence is a region, from -615 to -553 (SEQ ID NO: 4) containing an NFκβ region, in the region from -653 to -24 of the human matrix metalloproteinase-9 gene control region (SEQ ID NO: 1). More specifically, the HMMP9NFκβ inhibits the expression of the human matrix metalloproteinase-9 gene by binding to a 7-base region from -605 to -599 (tggaatt; SEQ ID NO: 5).

The present inventors synthesized Py-Im polyamides targeting a particular region in the human matrix metalloproteinase-9 gene promoter. The HMMP9 polyamides stably stayed in nuclei for 48 hours or longer without particularly disappearing. The polyamides exhibited more excellent permeability (at a low concentration without need of transfection medium) and higher stability in cultured breast cancer MDA-MB-231 cells than those of antisense oligonucleotide and ribozyme. The high permeability and stability of the polyamides provide an ideal drug delivery to the nuclei of eukaryotic cells for gene therapy.

Until recently, the development of Py-Im polyamide has been based on the structural characteristics of a transcription factor-DNA complex in a promoter sequence. The most efficient approach of targeting a sequence in a TATA box-containing promoter will be to design these polyamides to bind to base pairs adjacent to the TATA box. In most protein-encoding genes, the TATA box is located at 25-35 base pairs upstream of the transcription initiation site. Transcription-associated factor D (TAF_{II}D) contains a TATA box binding protein (TBP), which specifically binds to the TATA box, and forms a pre-initiation complex (PIC) by recruiting other transcription-related factors in the core promoter. The PIC initiates gene transcription and controls the expression of the gene through the interaction with an activator or a suppressor. Since TBP also binds to the minor groove of double helix DNA (Lee et al: Cell. 1991 Dec 20; 67 (6): 1241-50; Starr et al: Cell. 1991; 67: 1231-40; and Courey et al: Cell. 1988; 55: 887-98.), the synthetic polyamides occupy a binding site competitively with the TATA binding protein and interfere with the gene transcription. Of successful examples of polyamides designed based on various promoters, those targeting the TATA box are known to be always functional.

The upstream control region of the human matrix metalloproteinase-9 gene contains binding sites for a transcription factor activator protein-1 (hereinafter, also referred to as AP-1), NF-κβ, stimulatory protein-1 (hereinafter, also referred to as Sp-1) and a transcription factor Ets, and a GT box control region. *In-vitro* and *in-vivo* experimental models have demonstrated that the AP-1 protein plays an important role in the regulation of cell growth, differentiation, apoptosis and transformation (Sato et al: The Journal of Biological Chemistry 268 (1993) 23460-23468; Shaulian & Karin: Oncogene 20 (2001) 2390-2400; and Himelstein et al: Oncogene 14 (1997) 1995-1998). It has also been shown that the NF-κβ protein plays an important role in the regulation of the invasion, metastasis and drug resistance of tumor cells (Bond et al: FEBS Letters 435 (1998) 29-34; and Pahl: Oncogen 18 (1999) 6853-6866). It has further been shown that the GT box control region (GGGGTGGGG from -54 to -46; SEQ ID NO: 6) in MMP-9 plays an important role in MMP-9 expression and positively regulates the expression of MMP-9 through binding as a protein complex to a sequence from -71 to -30 in a transcription control region up to the upstream AP-1 binding region (TGAGTCA from -79 to - 73; SEQ ID NO: 7) (Sato et al, The Journal of Biological Chemistry 268 (1993) 23460-23468; and Farina et al: Cell Growth and Differentiation 10 (1999) 353-367).

Mutation in the binding sites described above may cause reduction or arrest of MMP-9 induction of the tissue plasminogen activator, and the inhibited expression of MMP-9 may cause reduction of the ability of tumor cells to invade or metastasize. Thus, the functional suppression of those transcription factors is an important target for the therapeutic purpose of inhibiting MMP expression and activity.

Mutation in the NF-κβ binding site or an NF-κβ decoy exhibited reduction in the MMP-9 induction of chemotherapeutic agents. The specific inhibition of tumor necrosis factor α, tissue plasminogen activator and NF-κβ bindings in the MMP-9 promoter presumably has no influence on the expression of MMP-9 in normal cells and the expression of other MMP genes.

The HMMP9AP1 of the present invention probably inhibits the bindings of the AP-1 protein and the GT box recognition complex by binding to a sequence containing the recognition site of a complex recognizing the AP-1 binding site and the GT box control region. This polyamide specifically reduced the expressions of MMP-9 mRNA and protein. The HMMP9NFκβ of the present invention probably inhibits the binding of the NF-κβ complex by binding to the NF-κβ binding site. This polyamide specifically reduced the expressions of hMMP-9 mRNA and protein.

In addition to regulation by the transcription factors in the promoter region, other factors are also likely to influence the gene expression. These factors encompass chromatin packing, polyadenylation, splicing, mRNA stability, translation initiation, etc. (Berger et al: Most Cell. 2001; 5: 263-8; McKeown Annu Rev Cell Biol. 1992; 8: 133-55; Decker et al: Trends Biochem Sci. 1994; 19: 336-40; and Kozak Annu Rev Cell Biol. 1992; 8: 197-225). Synthetic polyamide can approach the target site by the positioning of nucleosome and may affect a chromatin condensation/decondensation structure by targeting the specific sequence (Gottesfeld et al: J Mol Biol. 2002; 321: 249-63; and Gottesfeld et al: J Mol Biol. 2001; 309: 615-29). It has been demonstrated that pyrrole-imidazole polyamide opens heterochromatin brown satellite to allow GAF binding and as a result, causes change in phenotype in *Drosophilia melanogaster.* Pyrrole-imidazole polyamide can be synthesized relatively easily and designed to target a particular sequence. This pyrrole-imidazole polyamide may be used in the functional analysis of genomes and, ultimately, in gene therapy that inhibits the human matrix metalloproteinase-9 gene or inhibits or activates other related genes by changing their regulation.
The Py-Im polyamide according to the present invention can be designed based on an upstream region distant from the transcription initiation region, and shows inhibitory effect on the expression of the human matrix metalloproteinase-9 gene. Thus, the MMP9AP1 and MNW9NFκβ of the present invention can be used as a drug for inhibition of human matrix metalloproteinase-9 gene expression, treatment of disease related to this gene, and an anticancer agent.

Existing small molecules binding to a particular intracellular nucleotide sequence can be used as convenient test or evaluation drugs in the field of molecular cytology and are also probably useful as drugs for humans. The polyamide of the present invention can not only bind to *in-vitro* chromosomal DNA but also reach the nuclei of live cells through the outer membranes of the cells.

Metastasis consists of a multistage process including cell invasion and follows the next process of invasion cascade by repeating invasion of tumor cells into basement membranes, intravasation, extravasation, invasion into the tissues of distant organs, and intravasation. MMP-9 is overexpressed in various progressive or metastatic cancers. It has been hypothesized that the overexpressed MMP-9 degrades extracellular matrix containing cellular basement membranes to allow migration of tumor cells, leading to tumor invasion. Furthermore, the expression of MMP-9 is also important for neovascular endothelial cell growth, which induces the supply of nutrients to tumor vessels, and clinical tests had been conducted on diverse cancers using a large number of MMP molecular inhibitors for the purpose of inhibiting tumor invasion, metastasis and angiogenesis. However, myalgia and arthralgia with tendonitis had been found as adverse reactions in the early clinical tests. These adverse reactions are caused by the reasons that: the inhibitor does not work specifically for appropriate MMP; and the inhibitor also inhibits activity in normal cells. Thus, it is required that an inhibitor specific for a single MMP molecule should exert strong inhibitory effect, particularly, only during tumor invasion or induction of inflammation.

In this regard, "non-alkylated" Py-Im polyamide was designed to target human MMP-9 gene. MMP-9 plays an important role in tumor invasion or metastasis involving tumor growth, angiogenesis, etc., attributed to the degradation of cellular matrix, particularly, basement membranes.

The sustained high expression of MMP-9 is associated with the malignant transformations of many cancers including breast cancer. Particularly, it has been reported that the periodic stimulation of an MMP-9 promoter with NF-κβ or the binding of a transcription complex to GT box is involved in the malignant transformation of cancer. Moreover, a large number of *in-vitro* and *in-vivo* experimental models have also suggested that the AP-1 protein plays an important role in the regulation of cell growth, apoptosis and transformation.

Furthermore, the NF-κβ binding site in human MMP-9 may probably serve as a target of therapeutic resistance after chemotherapy or radiotherapy, in addition to tumor invasion and metastasis (Fukuyama et al: Molecular carcinogenesis 2007 46 402-4113). A large number of *in-vitro* and *in-vivo* experimental models have suggested that periodic stimulation with NF-κβ protein plays an important role in the regulation of the invasion, metastasis and drug resistance of tumor cells. The periodic stimulation with NF-κβ has been thought to participate in the regulation of expression of slow-reacting proteins involved in the shift from acute response to sustained inflammation reaction or chronic inflammation (Hoffmann et al: Science 2002 109 1241-1245). The binding of the Py-Im polyamide to the NF-κβ binding site probably inhibits this periodic stimulation by competition with binding of NF-κβ protein. From this, it is easily estimated that the Py-Im polyamide inhibits the sustained expression of MMP-9 mediated by the periodic stimulation with NF-κβ protein.

In gel shift analysis, the polyamide of the present invention exhibited strong and selective binding to the target DNA. Moreover, it was demonstrated that polyamide HMMP9AP1-FITC of the present invention is localized in the nuclei of breast cancer MDA-MB-231 cells *in vitro* after 45 minutes of addition and still localized in the nuclei even after 96 hours. It was also demonstrated that polyamide HMMP9NFκβ-FITC is localized in the nuclei of breast cancer MDA-MB-231 cells *in vitro* after 30 minutes of addition and still localized in the nucleic even after 96 hours. Thus, the polyamide of the present invention was shown to immediately migrate to nuclei *in vitro* and be specifically localized in the nuclei. Nucleic acid drugs such as antisense DNA, ribozyme and decoy have been developed as gene silencing agents. However, particularly, the decoy inhibits the binding of the target transcription factor in a manner similar to polyamide. However, the drugs are easily degraded by nuclease and therefore require a drug delivery system sufficient for arriving at tissues. On the other hand, the polyamide of the present invention is completely resistant to nuclease and therefore more suitable as a gene silencing drug. Thus, the HMMP9AP1 of the present invention migrates into the nuclei of cells, even without the use of approaches such as a drug delivery system, and is thus advantageous as a drug. This advantageous effect is advantageous for using the HMMP9AP1 and HMMP9NPκβ of the present invention in a human matrix metalloproteinase-9 gene inhibitor, treatment of disease related to this gene, and an anticancer agent.

The HMMP9AP1 and HMMP9NFκβ of the present invention inhibited the invasion activities of human breast cancer MDA-MB-231 cells and human uterine cervix cancer-derived HeLa cells in a polyamide concentration-dependent manner. Thus, the polyamides of the present invention can be used advantageously as an anticancer agent by inhibiting the migration of cancer cells.
Moreover, the HMMP9AP1 and HMMP9NFκβ of the present invention decreased the number of live cells of human breast cancer MDA-MB-231 cells and human uterine cervix cancer-derived HeLa cells in a concentration-dependent manner. This demonstrated that the polyamide of the present invention inhibited the growth of the cells.
Synthesized polyamides against sequences other than the nucleotide sequences targeted by the polyamide of the present invention (boxed sequences in Fig. 1) did not exhibit the inhibition of growth in growth inhibition assay using HeLa cells (data not shown). This demonstrated the specificity of the polyamide of the present invention.

The HMMP9AP1 and HMMP9NFκβ of the present invention inhibited the migrations of human breast cancer MDA-MB-231 cells and human uterine cervix cancer-derived HeLa cells in a concentration-dependent manner. Since migration is the first stage of invasion, it is important to evaluate inhibitory activity against the ability to migrate. Thus, the polyamide of the present invention can be used advantageously as an anticancer agent by inhibiting the migration, and by extension, invasion, of cancer cells.

The HMMP9AP1 and HMMP9NFκβ of the present invention remarkably inhibited the expressions of MMP-9 mRNA and MMP-9 protein in human breast cancer cells. This demonstrated that the polyamide of the present invention inhibits MMP-9 gene expression.
The HMMP91NFκβ of the present invention decreased the number and area of liver metastatic foci in mouse models with liver metastasis from human colon cancer.
Thus, the polyamide of the present invention can be used advantageously as a drug for inhibition of MMP-9 gene expression, a drug for treatment of MMP-9-related disease, specifically, disease involving angiogenesis, fibrosis or cell invasion, invasive tumor, metastatic tumor or tumor angiogenesis, and an anticancer agent.

### EXAMPLES

1. Synthesis of Py-Im polyamides corresponding to human promoter

### (1) Design of Py-Im polyamides corresponding to AP-1, GT box complex, and NFκβ binding sites of human matrix metalloproteinase-9 gene

### I. Materials and Methods

HMMP9AP1 and HMMP9NFκβ as described above were designed as Py-Im polyamides to bind to base pairs from -77 to -70 or from -605 to -599 in a human matrix metalloproteinase-9 promoter region.

### (2) Machine-assisted automatic synthesis of Py-Im polyamides using Fmoc method

The machine-assisted automatic synthesis of the pyrrole-imidazole polyamides was carried out using a continuous flow peptide synthesizer Pioneer (Trademark) (Applied Biosystems, Inc.) at 0.1 mmol scale (200 mg of Fmoc-β-alanine-CLEAR acid resin, 0.50 meq/g, Peptide Institute, Inc.). The automatic solid-phase synthesis consists of DMF washing, removal of Fmoc groups with 20% piperidine/DMF, methanol washing, coupling with monomers for 60 minutes in the presence of HATU and DIEA (4 equivalents each), ethanol washing, optional protection with anhydrous acetic acid/pyridine, and final DMF washing. The Py-Im polyamides were generally obtained in moderate yields (10-30%).

FITC coupling: A DMF solution containing a 4-fold excess of fluorescein (0.40 mmol) and DIEA (without HATU) dissolved therein was flushed for 60 minutes through a column.
General procedures: After removal of the Fmoc group of an Fmoc-β-alanine-Wang resin, the resin was continuously washed with methanol. A coupling step was carried out with an Fmoc amino acid, followed by washing with methanol These steps were repeated many times until the whole sequence was introduced. After the completion of the final coupling step, the N-terminal amino group was removed with piperidine, and 4 equivalents of fluorescein and DIEA were then added to the reaction container and reacted for 60 minutes. After the completion of the reaction, the reaction resin was washed and collected with DMF and methanol.

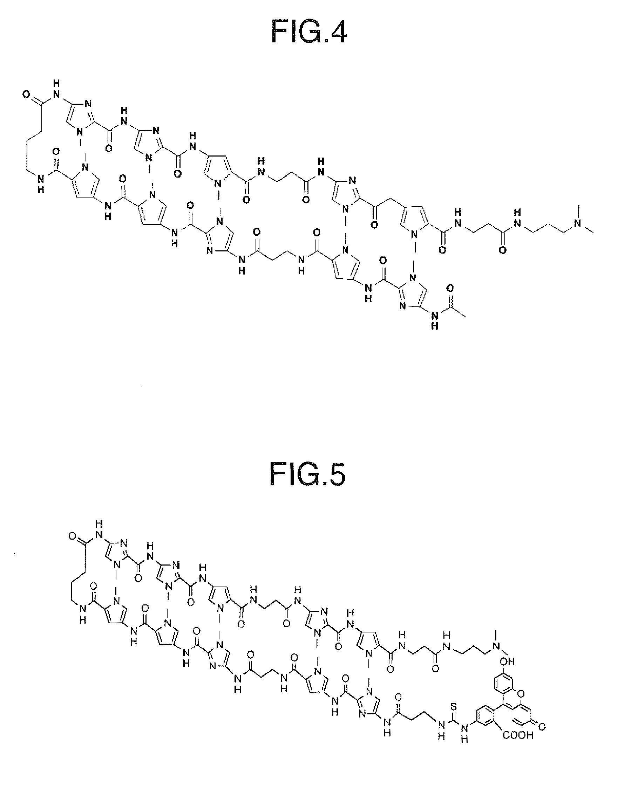

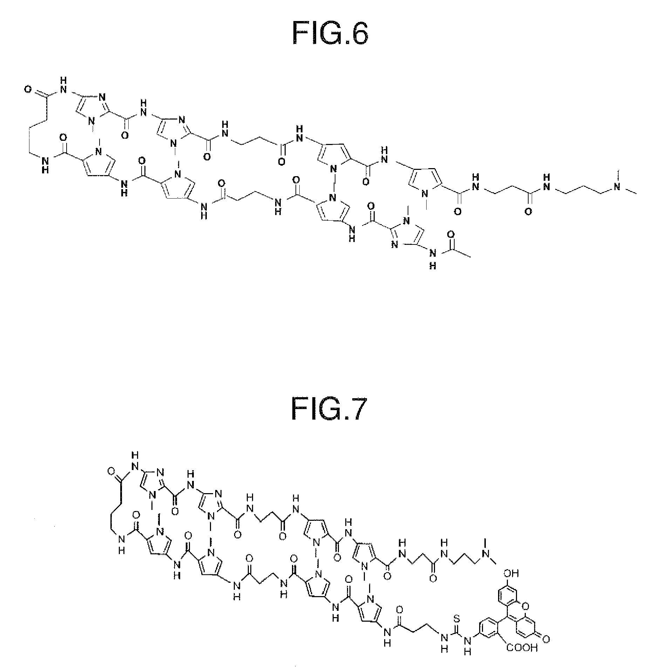

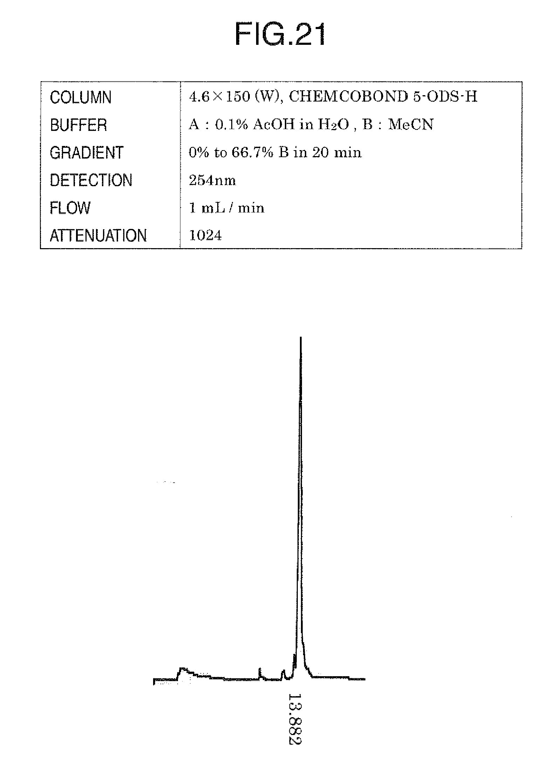

Degradation as carboxylic acid: The synthetic polyamides were isolated by precipitation with cold ethyl ether after a degradation step (5 ml of 91% TFA-3%/TIS-3% DMS-3% water mixture/0.1 mmol resin).
Degradation as amine: The synthetic polyamides were isolated by precipitation with cold ethyl ether after a degradation step (5 mL of N,N-dimethylaminopropyl amine/0.1 mmol resin, 50°C, overnight).
Purification: Final purification was carried out by analytical RP-HPLC at a flow rate of 10 mL/min using a linear gradient of buffer B (acetonitrile) in buffer A (0.1% TFA/water or 0.1% AcOH/water), with UV detection at 350 nm. HMMP9AP1, HMMP9AP1-FITC, HMMP9NFκβ and BMA4P9NFκβ-FITC are shown in Figs. 4, 5, 6 and 7, respectively. Moreover, their respective RP-HPLC charts are shown in Figs. 21, 22, 23 and 24.

### 2. Gel shift assay (Electromobility Shift Assay (EMSA))

### I. Materials and Methods

Oligonucleotides were synthesized and annealed to form 2 kinds of double-stranded oligonucleotides corresponding to the promoter base pairs against HMMP9AP1 and HMMP9NFκβ. One single-stranded oligonucleotide was labeled with FITC and hybridized with another single-stranded oligonucleotide as a complementary sequence to prepare double-stranded DNA. Specifically, nucleotides having the following nucleotide sequences were prepared: HMMP9AP1 (match) sense primer (5'GACCCCTGAGTCAGCACTTGCC) (SEQ ID NO: 8) corresponding to the region from -77 to -70 containing the AP-1 binding site; this sequence plus 2-base mutation, i.e., HMMP9AP1 oligo DNA (mismatch 1) sense primer (5'GACCCCTGAGTAGGCACTTGCC) (SEQ IT NO: 9); HMMP9NFκβ (match) sense primer (5'TGCCCCAGTGGAATTCCCCAGC) (SEQ ID NO: 10) containing the region from -605 to - 599 containing the NFκβ binding site; this sequence plus 2-base mutation, i.e., HMMP9NFκβ (mismatch 1) sense primer (5'TGCCCCAGTGGGGTTCCCCAGC) (SEQ ID NO: 11); and antisense primers respectively complementary to these 4 primers. 0.6 µM fluorescently labeled match double-stranded DNA or mismatch double-stranded DNA (double-stranded DNA prepared from the fluorescent sense primer and the antisense primer) was incubated with 10 µM polyamide or mismatch polyamide at 37°C for 1 hour in a binding buffer (40 mM Tris, pH 7.9, 250 mM NaCl, 25 mM EDTA, 25 mM DTT, 100 mM KCl). The obtained complexes were electrophoresed on a 20% polyacrylamide gel, and the mobilities of the fluorescently labeled double-stranded oligonucleotides were analyzed with a fluorescent image analyzer (FUJIFILM, LAS-4000, Tokyo, Japan).

### II. Results

### Binding of synthetic polyamides to double-stranded oligonucleotides

HMMP9AP1 and HMMP9NFκβ were studied for their bindings to target sequences by gel shift assay. 22-base sense and antisense oligonucleotides containing the target sequence of each pyrrole-imidazole polyamide were prepared and annealed to prepare double-stranded DNA of the target site, which was then incubated with the corresponding pyrrole-imidazole polyamide. The resulting complexes were electrophoresed on a polyacrylamide gel to study the bindings of the pyrrole-imidazole polyamides and their target sequences. The double-stranded DNA (DS) supplemented with the pyrrole-imidazole polyamide (Py-Im) HMNW9AP1 or HMMP9NFκβ exhibited lower mobility than that in the lanes containing only DS, suggesting polymerization. Thus, the binding between DS and Py-Im was demonstrated. The results are shown in Figs. 8 and 9.

### 3. Cell type and culture conditions

A human breast cancer MDA-MB-231 cell strain was used. The cells were cultured at 37°C in a humid environment containing 5% CO² in an RPMI1640 medium (Sigma-Aldrich Corp., St. Louis, MO, USA) supplemented with 100 µg/ml streptomycin, 100 units/ml penicillin and 10% fetal bovine serum (FBS) (Sigma-Aldrich Corp.). Human uterine cervix cancer HeLa cells were cultured in a Dulbecco's modified Eagle medium (DMEM, Invitrogen Life Technologies, Corp., Carlsbad, CA) containing 10% FBS and 2 mM L-glutamine,

### 4. Wound-healing migration assay

### I. Materials and Methods

To assay cellular migration in the process of wound healing, 3×10⁵ cells were inoculated to each well of an 8-well chamber slide, and the cell layer was damaged using a plastic micropipette when the cells reached confluence. The medium and debris were removed by suction, and 100 µl of a fresh medium containing various polyamides was replaced therefor. 48 hours after the damage, the cells were fixed with Diff-Quik solution (SYSMEX INTERNATIONAL REAGENTS CO., LTD., Kobe, Japan), then stained, and photographed using a phase-contrast microscope.

### II. Results

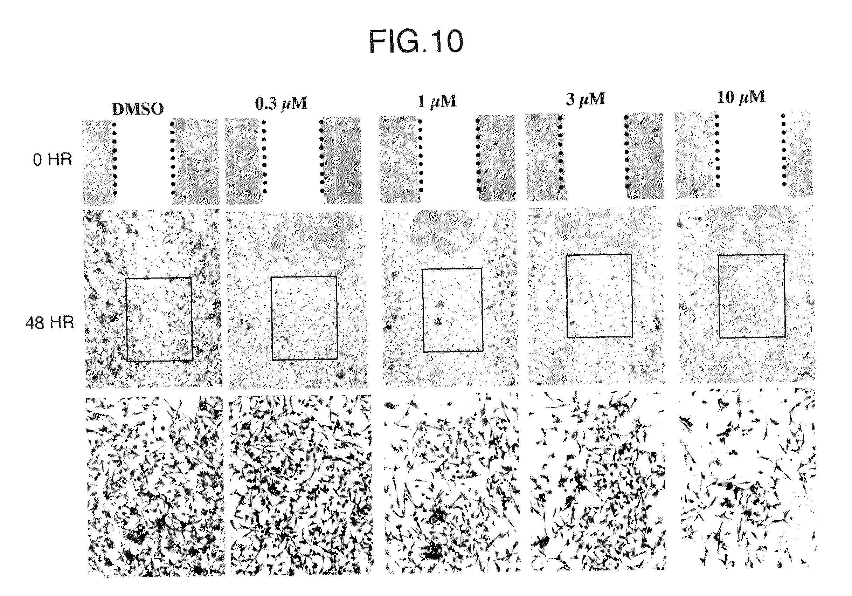

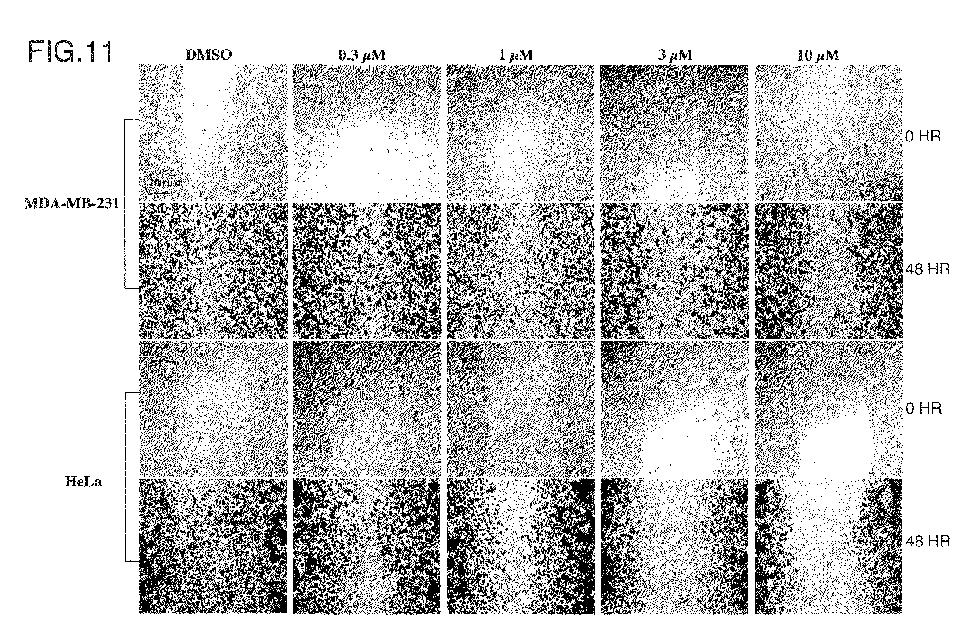

To evaluate the polyamides of the present invention for their abilities to migrate cells, the cells were treated with each polyamide at various concentrations. As a result of wound-healing migration analysis, the migration of MDA-MB-231 cells was shown to be inhibited by the polyamides of the present invention. The wounded region in DMSO-treated MDA-MB-231 was shown to be healed in 48 hours. In contrast, HMMP9AP1 and HMMP9NFκβ, the polyamides of the present invention, were shown to inhibit the migration of MDA-MB-231 cells in a concentration-dependent manner. Moreover, the HMMP9NFκβ was shown to inhibit the migration of HeLa cells in a concentration-dependent manner. These results demonstrated that the HMMP9AP1 and HMMP9NFκβ of the present invention inhibit the cellular migration of human breast cancer cells. Moreover, it was demonstrated that the HMMP9NFκβ also inhibits the cellular migration of human uterine cervix cancer cells. The results are shown in Figs. 10 and 11.

### 5. Invasion assay into matrigel

### I. Materials and Methods

Cell invasion assay was conducted using a 24-well cell culture BioCoat matrigel invasion chamber (BD Biosciences, Discovery Labware, Bedford, MA, USA). MDA-MB-231 cells (1 × 10³ cells/well) or HeLa cells (1 × 10³ cells/well) were suspended in an RPMI1640 or DEEM medium containing 0.1% FBS and added to the upper chamber. An RPMI1640 or DEEM medium containing 5% FBS was added to the lower chamber. Then, the cells were cultured at 37°C for 22 hours in a 5% CO₂ environment. Noninvasive cells remaining on the membrane were wiped off using a cotton swab. Invasion cells were fixed and stained with a Diff-Quik kit. The number of cells invading the membrane was determined in mean±standard deviation from a total of 3 membranes by counting in 10 fields of view per membrane using an optical microscope (200 × magnification).

### II. Results

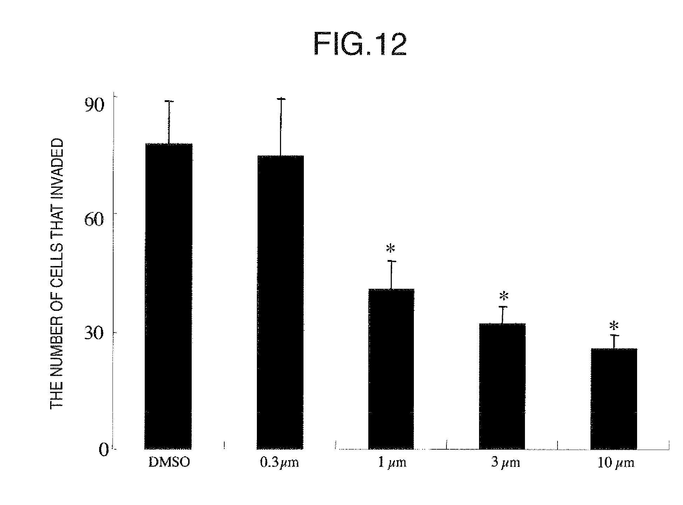

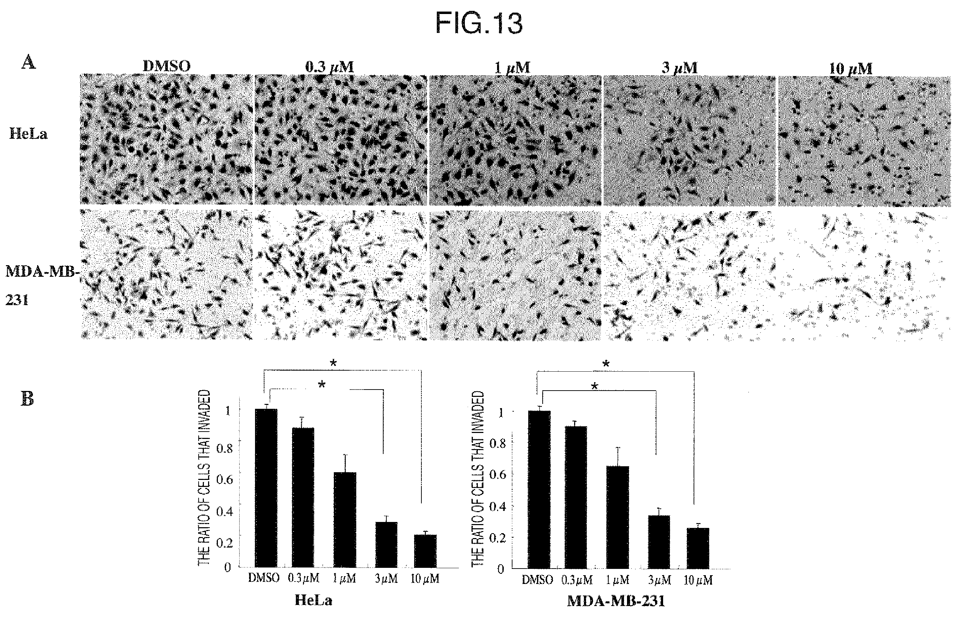

The degradation of constitutive proteins of extracellular matrix is important for the invasion of tumor cells. To evaluate the influence of HMMP9AP1 and HMMP9NFκβ, the MMP9 Py-Im polyamides of the present invention, on the invasion of tumor cells, 5 × 10³ MDA-MB-231 cells were incubated with each polyamide at various concentrations for 22 hours. The invasiveness of cells treated with DMSO or each polyamide of the present invention was compared in MDA-MB-231 cells. The DMSO-treated MDA-MB-231 cells remarkably invaded the transparent membrane coated with matrigel. This is suggested from the remarkably stained cells. In contrast, the cells treated with the HMMP9AP1 or HMMP9NFκβ of the present invention had much lower invasion into the membrane. This is suggested by staining intensity compared with the control. The MDA-MB-231 cells treated with 0.3 µM, 1 µM, 3 µM and 10 µM HMMP9AP1 exhibited 90%, 52%, 44% and 25.4% decreases, respectively, in invasion compared to the DMSO-treated control. The MDA-MB-231 cells treated with 0.3 µM, 1 µM, 3 µM and 10 µM HMMP9NFκβ exhibited 88%, 61%, 30% and 22% decreases, respectively, in invasion compared to the DMSO-treated control. These results demonstrated that the number of invasive cells that pass through matrigel decreases in a concentration-dependent manner.
The results are shown in Figs. 12 and 13.

### 6. Real-time RT-PCR assay (mRNA expression assay)

### I. Materials and Methods

MDA-MB-231 cells were treated with each polyamide of the present invention at various concentrations. 48 hours after the treatment, cell fractions were subjected to RNA separation using a TRIzol reagent (Invitrogen Life Technologies, Corp.) according to the manufacturer's manual. RNA was treated with DNase prior to cDNA synthesis. Complementary DNA strands were synthesized by reverse transcription using SuperScript^{™} First-Strand kit (Invitrogen Life Technologies, Corp.). In real-time RT-PCR (Thermal cycler Dice Real Time system TP800, Takara Bio Inc., Japan), SYBR Premix Ex Taq Kit (Takara Bio Inc.) was used. Primers for HMMP9AP1 (forward, 5'-GAGACCGGTGAGCTGGATAG-3', SEQ ID NO: 12; reverse 5'-TACACGCGAGTGAA.GGTGAG-3', SEQ ID NO: 13; 236 bp) and internal standard human endogenous glyceraldehyde-3-phosphate dehydrogenase (GADPH; forward, 5'-GCACCGTCAAGGCTGAGAAC-3', SEQ ID NO: 14; reverse, 5'-TGGTGAAGACGCCAGTGGA-3', SEQ ID NO: 15; 138 bp) were used. Moreover, primers for HMMP9NFκβ (forward, 5'-GAGACCGGTGAGCTGGATAG-3', SEQ ID NO: 16; reverse, 5'-TACACGCGAGTGAAGGTGAG-3', SEQ ID NO: 17; 236 bp) and internal standard human endogenous glyceraldehyde-3 -phosphate dehydrogenase (GADPH; forward, 5'-AGGCTGAGAAC-3', SEQ ID NO: 18; reverse, 5'-TGGTGAAGACGCCAGTGGA-3', SEQ ID NO: 19; 138 bp) were used. 25 µl of two-step RT-PCR mixture consists of 12.5 µl of SYBR Premix E_{X} TAq, 0.5 µl each of forward and reverse primers, 10.5 µl of RNase-free water and 1 µl of template cDNA. The real-time cycle conditions were performed by a reaction system involving 95°C for 10 seconds, 95°C for 5 seconds and 60°C for 30 seconds. The quantification of the MMP-9 gene was normalized by comparison with GAPDH expression.

### II. Results

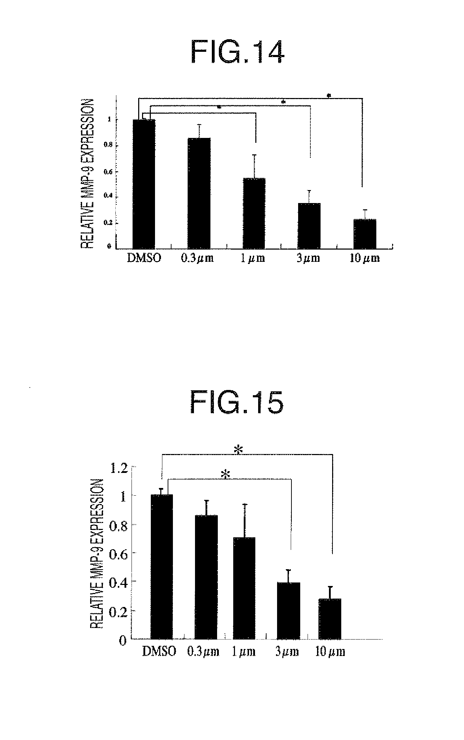

To further examine whether the cell migration and invasion activities derived from the polyamides of the present invention correlated with reduction in MMP-9 expression, MMP-9 expression levels were compared between MDA-MB-231 cells treated with each polyamide of the present invention and a DMSO-treated control by real-time RT-PCR. Transcriptional control plays an important role in MMP-9 expression. In the real-time RT-PCR analysis, the HMMP9AP1 decreased the expression level of MMP-9 mRNA by 84% at 0.3 µM, by 54% at 1 µM, by 38% at 3 µM and by 26% at 10 µM (Fig. 14). The HMMP9NFκβ decreased the expression level of MMP-9 mRNA by 86% at 0.3 µM, by 68% at 1 µM, by 40% at 3 µM and by 28% at 10 µM (Fig. 15). These results demonstrated that the polyamides of the present invention decrease MMP-9 mRNA levels in a concentration-dependent manner.

### 7. Zymography assay

### I. Materials and Methods

Proteins collected from culture supernatants of MDA-MB-231 cells were assayed using Novex (registered trademark) zymogram gelatin gel and XCell SureLock^{™} Mini-cell (Invitrogen Life Technologies, Corp.). The polyamides of the present invention were separately added at various concentrations to 2.5×10⁵ cells. After 48-hour incubation, the supernatants were collected. Proteins from each sample were mixed with Novex (registered trademark) Trisglycine SDS sample buffer (2x) and separated on a 10% zymogram gelatin gel. The gel was washed with a renaturing buffer and a developing buffer according to the manufacturer's manual. Then, the gel was digitized.

### II. Results

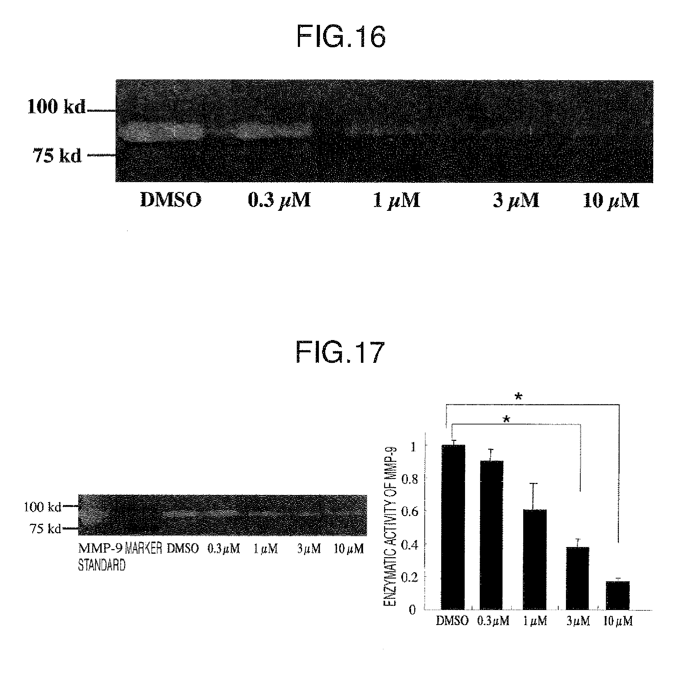

Protein activities in cell culture supernatants collected from cells treated for 48 hours were assayed. The treatment with 1µM HMMP9AP1 inhibited gelatin degradation activity by 50% compared with the control, and 10 µM HMMP9AP1 inhibited the enzymatic activity of MMP-9 almost completely (Fig. 16). The treatment with 1 µM HMMP9NFκβ inhibited gelatin degradation activity by 62%, and 10 µM HMMP9NFκβ inhibited it by 82% (Fig. 17).

### 8. Western blotting analysis (protein assay)

### I. Materials and Methods

MDA-MB-231 cells were treated with the polyamide of the present invention at various concentrations. 48 hours after the treatment, the cells were collected, and the total cell lysate was prepared into an extraction buffer containing a protease inhibition cocktail (Boehringer Ingelheim GmbH, Germany) containing 50 mM Tris-HCl, 150 mM NaCl, 10 mM EDTA and 1% Triton-X. Homogenates were centrifuged at 15,000xg at 4°C for 10 minutes. The supernatants were collected, and protein concentrations were measured. The protein sample (10 µg) was electrophoresed on NuPAGE+^{™} 10% Bis-Tris gel (Invitrogen Life Technologies, Corp.) and transferred to a PVDF (polyvinylidene difluoride) membrane (Millipore, Bedford, MA). The PVDF membrane was incubated overnight with rabbit anti-MMP-9 polyclonal antibodies (Calbiochem (registered trademark) Biosciences, Inc. La Jolla, CA) (1:500) obtained from Calbiochem (registered trademark). The PVDF membrane was washed three times with a Tris buffer containing 0.2% Tween 20. The immunoconjugated proteins were identified through reaction with peroxidase-conjugated goat antibodies against rabbit IgG (MP Biomedicals, Inc.) and subsequently amplified by chemiluminescence detection (Amersham, Piscataway, NJ).

### II. Results

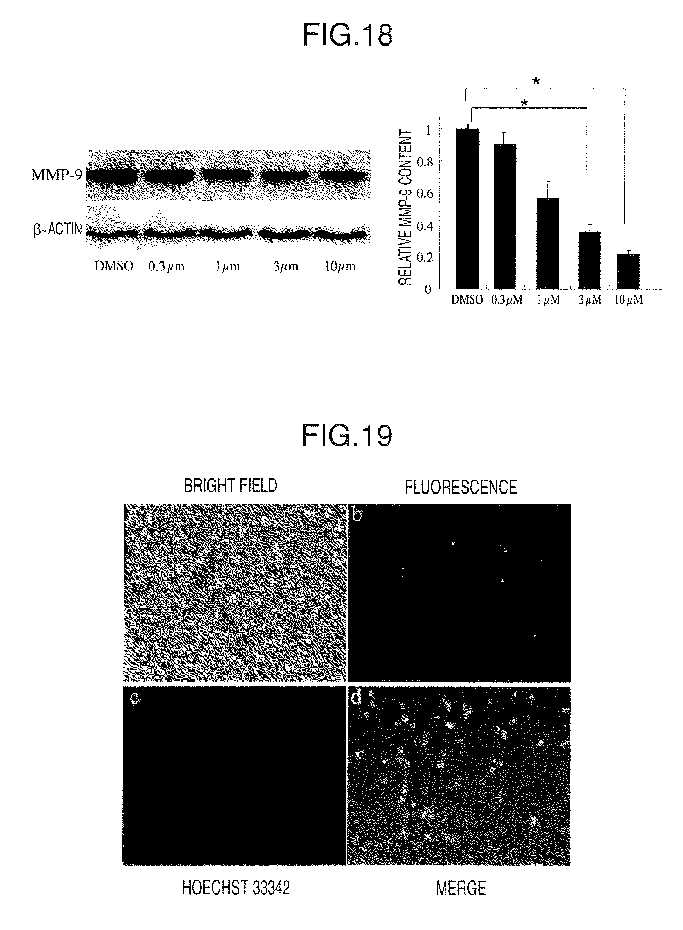

To examine whether the polyamide of the present invention inhibited MMP-9 protein expression, western blotting analysis was conducted. MMP-9 expression levels were compared between MDA-MB-231 cells treated with the polyamide of the present invention and a DMSO-treated control. Transcriptional control plays an important role in MMP-9 protein expression. The western blotting analysis showed that the expression of MMP-9 protein in the MDA-MB-231 cells treated with HMMP9NFκβ decreased in an HMMP9NFκβ concentration-dependent manner. The results are shown in Fig. 18.

### 9. In-vitro distribution of fluorescently labeled PIP

### I. Materials and Methods

MDA-MB-231 cells were inoculated at a concentration of 3.0×10⁴ cells/well to a 6-well plate. The cells were cultured at 37°C in a 5% CO² environment in 2 ml of RPMI1640 medium containing 10% FBS (Invitrogen), After 24-hour culture, fluorescently labeled polyamide HMMP9AP1 (hereinafter, also referred to as HMMP9AP1-FITC) and fluorescently labeled polyamide HMMP9NFκβ (hereinafter, also referred to as HMMP9NFKκβ-FITC) were separately added at a final concentration of 10 µM to a growth medium of the MDA-MB-231 cells, and culture was further continued for 2 hours. The cells were washed, and an FBS-free medium was added thereto. Live cells were observed at a 200× magnification at the predetermined times (30 minutes and 96 hours) and fixed in 4% paraformaldehyde for 10 minutes. The nuclei were stained with Hoechst 33342 (Invitrogen Life Technologies, Corp., Carlsbad, CA), followed by observation again.

### II. Results

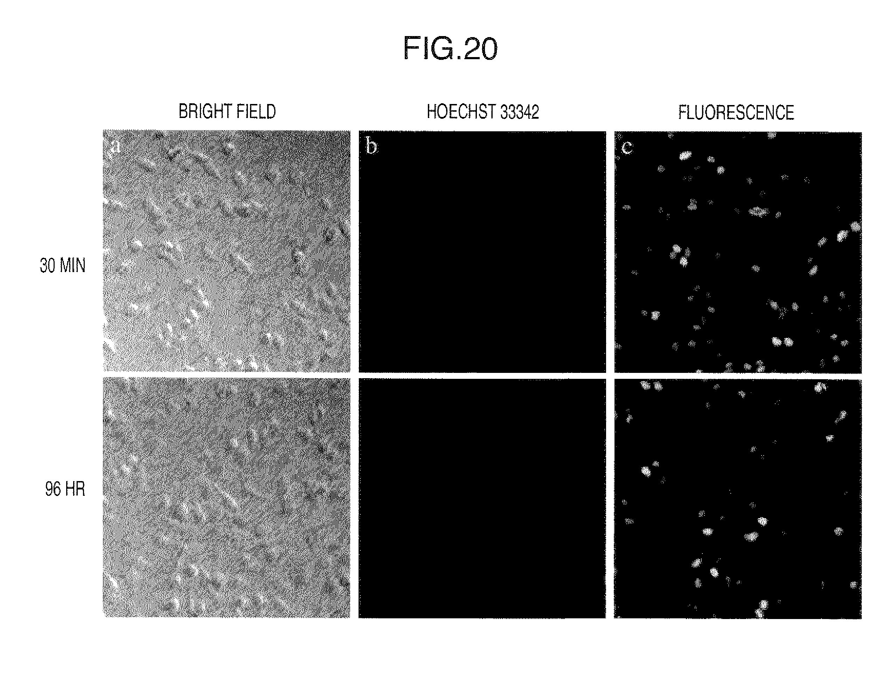

The HMMP9AP1-FITC was observed to be localized in the nucleic of all the MDA-MB-231 cells after 45 minutes of addition to the growth medium for cell culture (data not shown). Even after 96 hours from the addition, the HMMP9AP1-FITC was confirmed to be stably present in the nuclei. Moreover, the HMMP9NFκβ-FITC was observed to be localized in the nucleic of all the cells after 30 minutes of addition. Even after 96 hours from the addition, the HMMP9NFκβ-FITC was confirmed to be stably present in the nuclei. The results are shown in Figs. 19 and 20.

### 10. Growth inhibition assay using MDA-MB-231 cells and HeLa cells I. Materials and Methods

Cell growth inhibition test was conducted using MDA-MB-231 and HeLa cells. The HeLa cells were inoculated at a concentration of 2500 cells/well to a 96-well microtiter plate and incubated for 24 hours in 100 µl of Dulbecco's modified Eagle medium (DMEM) containing 10% fetal bovine serum (Invitrogen). Then, after medium replacement, the cells were cultured for 72 hours in a medium supplemented with 10% fetal bovine serum containing 0 µM to 30 µM (0 µM, 0.3 µM, 1 µM, 3 µM, 10 µM and 30 µM) polyamide HMMP9NFKκβ, and 10 µl of live cell counting reagent SF (Nacalai Tesque, Inc.) was added thereto, followed by 2-hour color reaction. Absorbance was measured at 450 nm using an ARVO microtiter plate reader. This experiment was conducted by modified MTT assay (WST-8^{™}: Nacalai Tesque, Inc.) using water-soluble formazan,

### II. Results

The addition of the polyamide HMMP9NFκβ to MDA-MB-231 cells and HeLa cells decreased the number of live cells in a concentration-dependent manner. Such results suggest that the polyamide of the present invention is effective as an anticancer agent. The results are shown in Fig. 25.

### 11. Immunoprecipitation/PCR assay with anti-NF-κβ antibodies

### 1. Materials and Methods

Immunoprecipitation/PCR assay with anti-NF-κβ antibodies was conducted using MDA-MB-231 and HeLa cells. The MDA-MB-231 and HeLa cells were separately inoculated to a 100-mm culture dish and cultured for 24 hours in 100 µl of Dulbecco's modified Eagle medium (DMEM) containing 10% fetal bovine serum (Invitrogen) and then with 1 µM match polyamide HMMP9NFκβ or 1 µM mismatch polyamide (shown in Fig. 26) for 4 hours. Protein-DNA complexes were fixed in 1% formaldehyde, reacted with specific NF-κB p65 antibodies and nonspecific rabbit immunoglobulin G antibodies, and collected by precipitation as antibody-protein-DNA complexes using Protein A beads. From the protein-DNA complexes, DNA was separated through reaction with 5 M NaCl and subjected to amplification reaction using PCR primers flanking the NFκβ binding sequence of MMP9. The NFκβ-bound DNA of an MMP9 gene promoter region is identified as a PCR product.
An MMP-9 promoter region from -657 to -484 was amplified using PCR primers (forward: 5'-TGTCCCTTTACTGCCCTGA-3' (SEQ ID NO: 20) and reverse: 5'-ACTCCAGGCTCTGTCCTCCTCTT-3' (SEQ ID NO: 21)) (Schwingshackl A, Duszyk M, Brown N, Moqbel R. HµMan eosinophils release matrix metalloproteinase-9 on stimulation with TNF-α. J Allergy Clin Immunol 104: 983-989, 1999).

### II. Results

The NF-κβ region of the MMP-9 promoter was immunoprecipitated with anti-NF-κβ p65 antibodies, when the cells were untreated with the polyamide of the present invention or treated with the mismatch polyamide. However, the NF-κβ p65-NF-κβ binding region complex was not detected in the immunoprecipitation assay, when the cells were treated with the polyamide MMP9NFκβ of the present invention. The results are shown in Fig. 27. These results show that the polyamide MMP9NFκβ of the present invention inhibited *in vivo* the specific binding of NF-κβ protein with the MMP-9 promoter region.

### 12. Tissue distribution of fluorescently labeled polyamide HMMP9Fκβ

### I. Materials and Methods

0.15 mg of fluorescently labeled polyamide HMMP9NFκβ (hereinafter, also referred to as HMMP9NFκβ-FITC) was injected at a final concentration of 7.5 mg/kg to the tail veins of mice. On day 1 (24 hours), day 6 and day 21 after the intravenous injection, the mice were dissected, and each tissue of the livers, the spleens and the kidneys was collected and prepared into frozen sections, which were then fixed in 4% paraformaldehyde for 30 minutes. The nuclei were stained with Hoechst 33342 (Invitrogen Life Technologies, Corp., Carlsbad, CA), followed by observation.

### II. Results

A large number of the FITC-labeted polyamides HMMP9NFκβ were incorporated in the nuclei of liver and kidney cells after 1 day of administration. They were also incorporated in the nuclei of spleen cells. Moreover, even after 6 days after administration, strong FITC fluorescence was observed. Furthermore, the little attenuation of FITC fluorescence intensity in 6 days suggested that the polyamide HMMP9NFκβ is very stable in the nuclei of the cells. However, after 21 days, FITC fluorescence intensity remarkably attenuated in the cells of each tissue, indicating that DNA binding in the nuclei of the cells gradually disappears. This is a phenomenon that can be explained by the urinary or biliary excretion of the polyamide, as with other polyamide compounds. The results are shown in Fig. 28.

### 13. Evaluation using immunodeficient athymic mouse (nude mouse) models with liver metastasis from human colon cancer

### I. Materials and Methods

5-week-old male nude mice (BALB/c Nude Mice CBy.Cg-Foxn1 nu/J) (Jackson Laboratory) were purchased, maintained in a pathogen-free environment until 6 week olds, and used in the experiment. A human colon cancer cell strain HT29 was monolayer-cultured in a 100-cm² cell culture flask and treated with 0.05% trypsin and 0.02% EDTA solutions before reaching confluence. The cells were collected, centrifuged at 800 rpm for 3 minutes, then washed with PBS containing neither Ca²⁺ nor Mg²⁺, and cultured for 1 hour in a culture solution adjusted to a polyamide concentration of 10 µM or a polyamide-free culture solution. Then, a cell suspension having a concentration of 3×10⁶ cells in 100 µl again was prepared using PBS. The thus-prepared 3 groups of the cells containing HMMP9NFκβ polyamide, containing mismatch polyamide and in polyamide-free PBS were separately administered to the nude mice (6 individuals/group). Longitudinal incision was made in the skin on the left side of the abdominal regions of the nude mice under anesthesia. After peritoneotomy to the spleen, 100 µl of the colon cancer cell suspension was transplanted to the splenic subcapsular region using a 29-G injection needle (Fig. 29A). The reliable splenic subcapsular transfer of the cell grafts was confirmed based on change in the color tone of the cell-transplanted site through the splenic capsule and the absence of bleeding or leakage of the suspension. After the transplantation, the mice were raised for 6 weeks and subjected to autopsy. The livers and the spleens were extracted, fixed in a 10% buffered formalin solution, then histologically stained with hematoxylin eosin, and subjected to microscopic observation. Fig. 29B shows the number of metastatic foci in each group. The liver metastatic foci are circled in the preparations.

### II. Results

In all the cases, the invasion of colon cancer cells was observed as primary foci in the transplanted site in the spleens and as metastatic foci in the livers. As shown in Fig. 29(B), the number and size of liver metastatic foci were distinctly small in the HMMP9NFκβ group, and the area thereof was also small therein. By contrast, the size of metastatic foci was large in the polyamide-untreated group and the mismatch polyamide-administered group, and these groups were observed to have the strong tendency of metastatic foci to fuse with each other and a large number of metastatic foci (Fig. 29). This probably indicated that the HMMP9NFκβ polyamide inhibited the liver metastasis of human colon cancer transplanted in the spleens of the nude mice.

### 14. Statistical analysis

The results were indicated in mean±SE. Statistical significance was evaluated by Student's t-test. When the p value is less than 0.05, the results were determined to be significant.

### INDUSTRIAL APPLICABILITY

The present invention relates to a matrix metalloproteinase-9 gene expression inhibitor, a therapeutic drug for matrix metalloproteinase-9-related disease, and an anticancer agent. More specifically, the present invention relates to a drug comprising pyrrole-imidazole polyamide having a specific structure.

### FREE TEXT IN SEQUENCE LISTING

SEQ ID NO: 8 Sense primer
SEQ ID NO: 9 Sense primer
SEQ ID NO: 10 Sense primer
SEQ ID NO: 11 Sense primer
SEQ ID NO: 12 Forward primer
SEQ ID NO: 13 Reverse primer
SEQ ID NO: 14 Forward primer
SEQ ID NO: 15 Reverse primer
SEQ ID NO: 16 Forward primer
SEQ ID NO: 17 Reverse primer
SEQ ID NO: 18 Forward primer
SEQ ID NO: 19 Reverse primer
SEQ ID NO: 20 Forward primer
SEQ ID NO: 21 Reverse primer

## Claims

1. A medicament comprising pyrrole-imidazole polyamide containing an N-methylpyrrole unit (hereinafter, also referred to as Py), an N-methylimidazole unit (hereinafter, also referred to as Im) and a γ-aminobutyrate unit, wherein the pyrrole-imidazole polyamide can be folded at the y-aminobutyrate unit into a U-shaped conformation in a minor groove of a double helix region (hereinafter, referred to as a target region) which comprises a portion or the whole of a nucleotide sequence from -84 to -24 (SEQ ID NO: 2) in a human matrix metalloproteinase-9 (hereinafter, also referred to as hMMP-9) gene promoter, and a strand complementary thereto, wherein a Py/Im pair corresponds to a C-G base pair, an Im/Py pair corresponds to a G-C base pair, and a Py/Py pair corresponds to both an A-T base pair and a T-A base pair.

2. The medicament according to claim 1, further comprising a β-alanine unit.

3. The medicament according to claim 1, further comprising a fluorescein isothiocyanate unit.

4. The medicament according to any one of claims to 3, wherein the medicament is intended for inhibition of human matrix metalloproteinase-9 gene expression.

5. The medicament according to any one of claims 1 to 3, wherein the medicament is intended for treatment of human matrix metalloproteinase-9-related disease.

6. The medicament according to claim 5, wherein the medicament is intended for use as an anticancer agent.

7. The medicament according to any one of claims 1 to 6, wherein the target region is a double helix region which comprises a portion or the whole of a nucleotide sequence from - 77 to -70 (SEQ ID NO: 3) in the human matrix metalloproteinase-9 promoter, and a strand complementary thereto.

8. The medicament according to any one of claims to 7, wherein the pyrrole-imidazole polyamide is represented by the following formula:

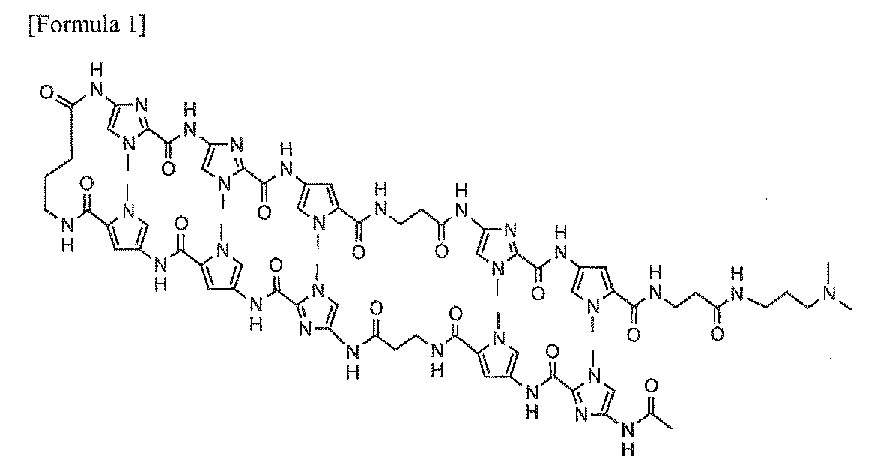

9. Pyrrole-imidazole polyamide represented by the following formula:

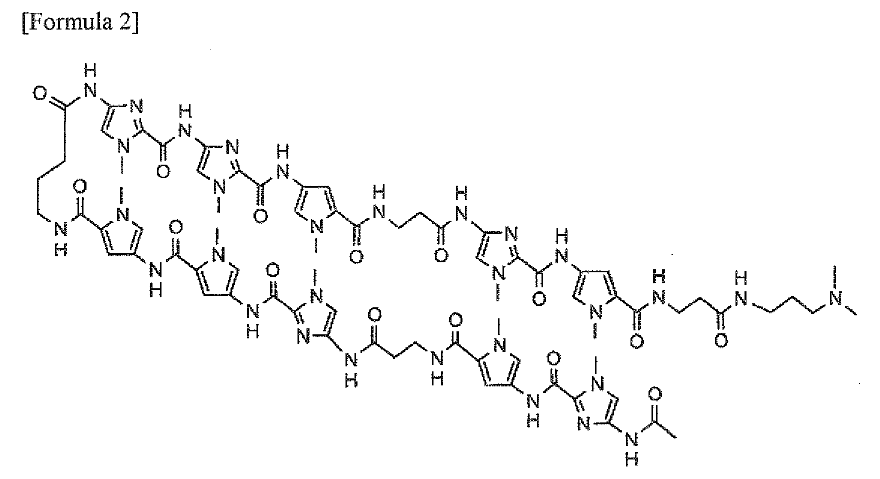

10. A medicament comprising pyrrole-imidazole polyamide containing an N-methylpyrrole unit, an N-methylimidazole unit and a γ-aminobutyrate unit, wherein the pyrrole-imidazole polyamide can be folded at the γ-aminobutyrate unit into a U-shaped conformation in a minor groove of a double helix region which comprises a portion or the whole of a nucleotide sequence from -615 to -553 (SEQ ID NO: 4) in a human matrix metalloproteinase-9 gene promoter, and a strand complementary thereto, wherein a Py/Im pair corresponds to a C-G base pair, an Im/Py pair corresponds to a G-C base pair, and a Py/Py pair corresponds to both an A-T base pair and a T-A base pair.

11. The medicament according to claim 10, further comprising a β-alanine unit.

12. The medicament according to claim 10, further comprising a fluorescein isothiocyanate unit.

13. The medicament according to any one of claims 10 to 12, wherein the medicament is intended for inhibition of human matrix metalloproteinase-9 gene expression.

14. The medicament according to any one of claims to to 12, wherein the medicament is intended for treatment of human matrix metalloproteinase-9-related disease.

15. The medicament according to claim 14, wherein the medicament is intended for use as an anticancer agent.

16. The medicament according to any one of claims 10 to 15, wherein the target region is a double helix region which comprises a portion or the whole of a nucleotide sequence from -605 to -599 (SEQ ID NO: 5) in the human matrix metalloproteinase-9 promoter, and a strand complementary thereto.

17. The medicament according to any one of claims 10 to 16, wherein the pyrrole-imidazole polyamide is represented by the following formula:

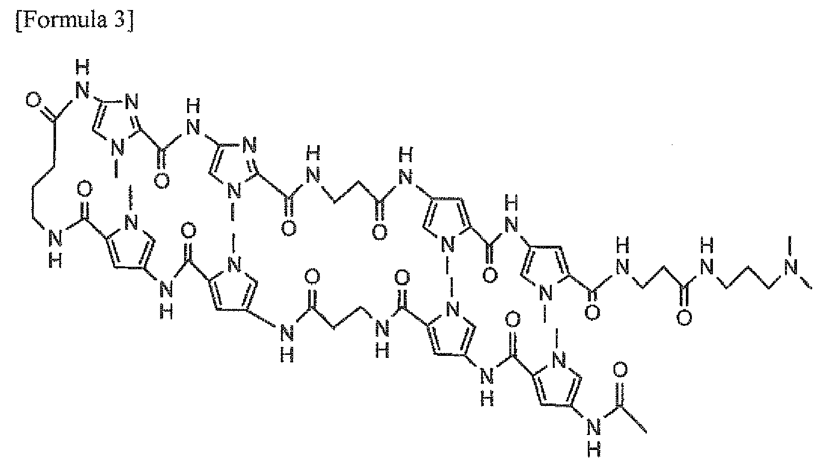

18. Pyrrole-imidazole polyamide represented by the following formula:

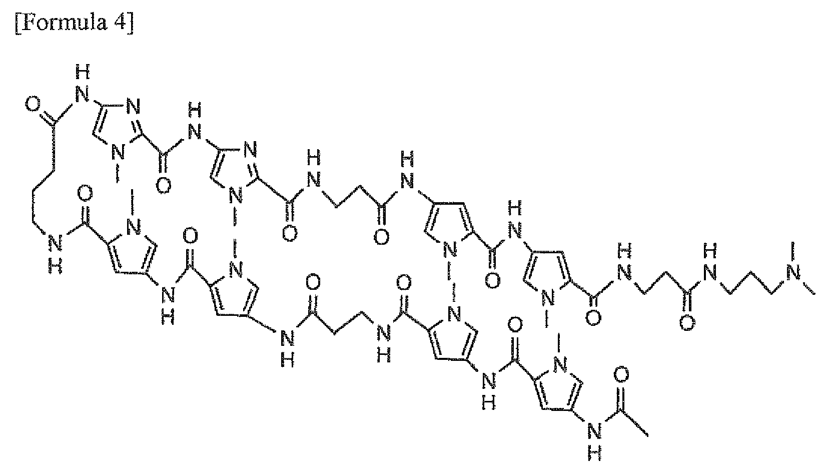

19. A pharmaceutical composition comprising a medicament according to any one of claims 1 to 8 or a medicament according to any one of claims 10 to 17 and a pharmaceutically acceptable carrier.

20. A medicament comprising pyrrole-imidazole polyamide according to claim 9 or 18 and a pharmaceutically acceptable carrier.

21. Pyrrole-imidazole polyamide represented by the following formula:

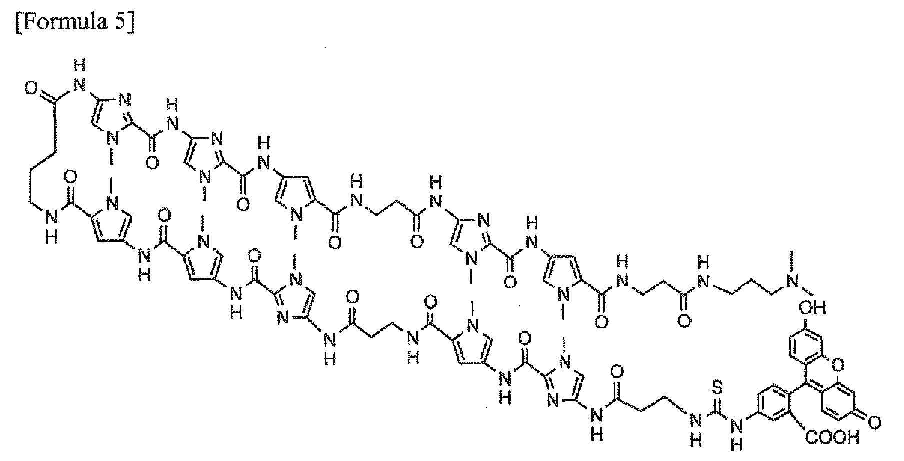

22. Pyrrole-imidazole polyamide represented by the following formula:

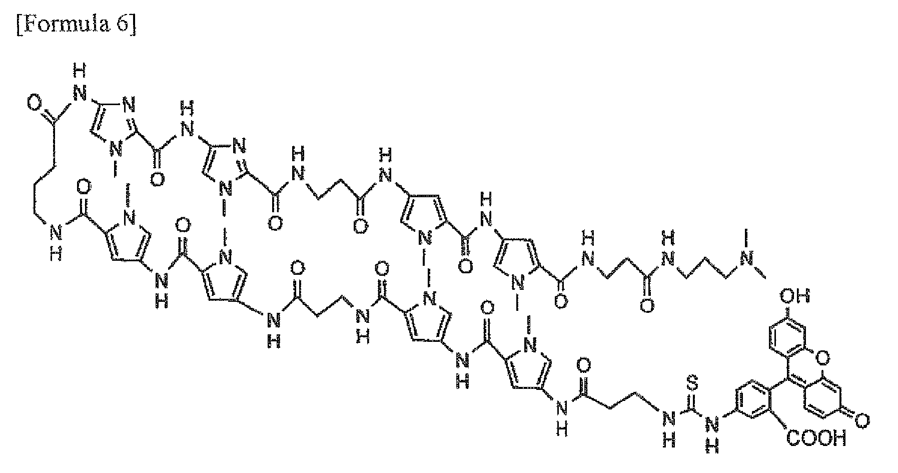

23. The pyrrole-imidazole polyamide according to claim 9, 18, 21 or 22, wherein the pyrrole-imidazole polyamide is used as a reagent of a basic experiment.
